# EUROPÄISCHE PATENTANMELDUNG

(11) **EP 0 705 834 A1**
(43) Veröffentlichungstag der Anmeldung: **10.04.1996**
(21) Anmeldenummer: 95810470.5
(22) Anmeldetag: 18.07.1995
(51) Int. Cl.: C07D 487/06, A61K 31/495

(54) **Azaaliphatisch Überbrückte Chinoxalin-2,3-dione**

(30) Priorität: 27.07.1994 CH 2366/94
(71) Anmelder: CIBA-GEIGY AG, CH-4002 Basel (CH)
(72) Erfinder: Moretti, Robert, Dr., CH-4123 Allschwil (CH); Zimmermann, Kaspar, Dr., CH-4125 Riehen (CH)

(57) **Zusammenfassung**

Azaaliphatisch überbrückte Chinoxalin-2,3-dione der Formel I 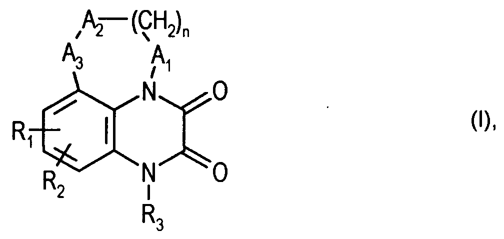
worin A₁ Niederalkyliden oder eine Gruppe der Formel >CH-A₄-R₄ (Ia) darstellt, A₂ Niederalkyliden oder eine Gruppe der Formeln >CH-A₄-R₄ (Ia) oder >C=O (Ib) oder >CH(OH)-A₅-R₄ (Ic) bedeutet A₃ eine Gruppe der Formel >N-A₆-R₅ (Id) darstellt, A₄ für Niederalkylen steht, A₅ und A₆ unabhängig voneinander Niederalkylen oder eine direkte Bindung darstellen, n für 0 oder 1 steht,
R₁ und R₂ unabhängig voneinander Wasserstoff, gegebenenfalls durch Niederalkyl und/oder Niederalkanoyl substituiertes Amino, Nitro, Niederalkanoyl, gegebenenfalls verethertes Hydroxy, gegebenenfalls verestertes Carboxy, Carbamoyl, Cyano, gegebenenfalls halogeniertes Niederalkyl oder Halogen bedeuten, R₃ Wasserstoff oder Hydroxy bedeutet, R₄ gegebenenfalls verestertes oder amidiertes Carboxy, gegebenenfalls verestertes Phosphono oder 5-Tetrazolyl darstellt und R₅ Wasserstoff, Niederalkyl, Niederalkenyl, Niederalkinyl, Aryl, Niederalkanoyl, gegebenenfalls verestertes oder amidiertes Carboxy, Cyano, 5-Tetrazolyl, gegebenenfalls verethertes oder verestertes Hydroxy oder Dihydroxyniederalkyl bedeutet, und ihre Salze weisen antagonistische Eigenschaften gegenüber excitatorischen Aminosäuren auf und können als antikonvulsive und anti-neurodegenerative Arzneimittelwirkstoffe verwendet werden.

## Beschreibung

Die Erfindung betrifft neue azaaliphatisch überbrückte Chinoxalin-2,3-dione der Formel I 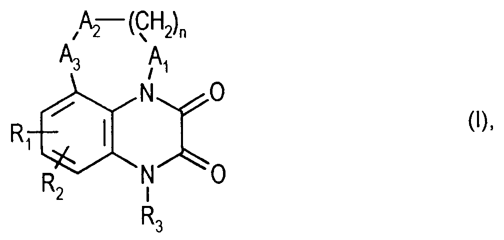
worin
A₁ Niederalkyliden oder eine Gruppe der Formel >CH-A₄-R₄ (Ia) darstellt,
A₂ Niederalkyliden oder eine Gruppe der Formeln >CH-A₄-R₄ (la) oder >C=O (Ib) oder >CH(OH)-A₅-R₄ (Ic) bedeutet,
A₃ eine Gruppe der Formel >N-A₆-R₅ (Id) darstellt,
A₄ für Niederalkylen steht,
A₅ und A₆ unabhängig voneinander Niederalkylen oder eine direkte Bindung darstellen,
n für 0 oder 1 steht,
R₁ und R₂ unabhängig voneinander Wasserstoff, gegebenenfalls durch Niederalkyl und/oder Niederalkanoyl substituiertes Amino, Nitro, Niederalkanoyl, gegebenenfalls verethertes Hydroxy, gegebenenfalls verestertes Carboxy, Carbamoyl, Cyano, gegebenenfalls halogeniertes Niederalkyl oder Halogen bedeuten,
R₃ Wasserstoff oder Hydroxy bedeutet,
R₄ gegebenenfalls verestertes oder amidiertes Carboxy, gegebenenfalls verestertes Phosphono oder 5-Tetrazolyl darstellt und
R₅ Wasserstoff, Niederalkyl, Niederalkenyl, Niederalkinyl, Aryl, Niederalkanoyl, gegebenenfalls verestertes oder amidiertes Carboxy, Cyano, 5-Tetrazolyl, gegebenenfalls verethertes oder verestertes Hydroxy oder Dihydroxyniederalkyl bedeutet,
und ihre Salze, die neuen Verbindungen enthaltende pharmazeutische Präparate und ihre Verwendung als Arzneimittelwirkstoffe.

Gegebenenfalls durch Niederalkyl und/oder Niederalkanoyl substituiertes Amino ist beispielsweise Amino, Niederalkylamino, Niederalkanoylamino, Diniederalkylamino oder N-Niederalkyl-N-niederalkanoyl-amino.

Gegebenenfalls verethertes oder verestertes Hydroxy ist beispielsweise gegebenenfalls mit einem Niederalkanol verethertes oder mit einer Niederalkansäure verestertes Hydroxy, insbesondere Hydroxy, Niederalkanoyloxy oder Niederalkoxy, kann aber auch Niederalkenyloxy oder Niederalkinyloxy sein.

Gegebenenfalls halogeniertes Niederalkyl ist beispielsweise Niederalkyl oder Polyhalogenniederalkyl, insbesondere Trifluormethyl.

Gegebenenfalls verestertes oder amidiertes Carboxy ist beispielsweise Carboxy, Niederalkoxycarbonyl, Carboxyniederalkoxycarbonyl, Niederalkoxycarbonylniederalkoxycarbonyl, Niederalkanoyloxyniederalkoxycarbonyl, unsubstituiertes oder durch Niederalkyl, Niederalkoxy, Hydroxy, Halogen und/oder Trifluormethyl substituiertes Phenylniederalkoxycarbonyl, Carbamoyl, Niederalkylcarbamoyl, Hydroxyniederalkylcarbamoyl, Diniederalkylcarbamoyl, Diniederalkylaminoniederalkylcarbamoyl, Aminoniederalkylaminoniederalkylcarbamoyl, 2-Oxoimidazolidin-1-ylniederalkylcarbamoyl, gegebenenfalls durch Aminoniederalkylamino bzw. 2-Oxoimidazolidin-1-yl substituiertes Niederalkylenaminoniederalkylcarbamoyl, wie Aminoniederalkylaminoniederalkylencarbamoyl oder 2-Oxoimidazolidin-1-ylnideralkylencarbamoyl, Oxaniederalkylenaminoniederalkylcarbamoyl, gegebenenfalls durch Carboxy oder Niederalkoxycarbonyl substituiertes Cycloalkylcarbamoyl, Cycloalkylniederalkylcarbamoyl, unsubstituiertes oder durch Niederalkyl, Niederalkoxy, Hydroxy, Halogen und/oder Trifluormethyl substituiertes Phenylniederalkylcarbamyol, unsubstituiertes oder durch Niederalkyl, Niederalkoxy, Hydroxy, Halogen, Nitro, Carboxy, Niederalkoxycarbonyl, Phenyl, Phenyloxy und/oder Trifluormethyl substituiertes N-Phenyl- oder N-Niederalkyl-N-phenylcarbamoyl oder gegebenenfalls verethertes N-Hydroxycarbamoyl, wie N-Hydroxycarbamoyl, N-Niederalkoxycarbamoyl, N-Niederalkenyloxycarbamoyl oder unsubstituiertes oder durch Niederalkyl, Niederalkoxy, Hydroxy, Halogen und/oder Trifluormethyl substituiertes N-Phenylniederalkoxy oder N-Phenylniederalkenyloxycarbamoyl.

Gegebenenfalls verestertes Phosphono kann vollständig oder partiell verestert sein und bedeutet beispielsweise Phophono, Niederalkylphosphono, Diniederalkylphosphono oder Triniederalkylphosphono.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Aminoniederalkylaminoniederalkylcarbamoyl ist beispielsweise N-(Amino-C₂-C₄-alkylamino-C₁-C₄-alkyl)carbamoyl, wie N-[2-(2-Aminoethylamino)ethyl]carbamoyl.

Aminoniederalkylaminoniederalkylencarbamoyl ist beispielsweise Amino-C₂-C₄-alkylaminopiperidinocarbonyl, wie 4-(2-Aminoethylamino)piperidinocarbonyl.

Carboxyniederalkoxycarbonyl ist beispielsweise Carboxy-C₁-C₄-alkoxycarbonyl, wie Carboxymethoxycarbonyl, 2-Carboxyethoxycarbonyl, 3-Carboxypropyloxycarbonyl oder 4-Carboxybutyloxycarbonyl.

Cycloalkylcarbamoyl ist beispielsweise N-C₃-C₆-Cycloalkylcarbamoyl, wie Cyclopropylcarbamoyl, Cyclobutylcarbamoyl, Cyclopentylcarbamoyl oder Cyclohexylcarbamoyl, kann aber auch polycyclisches Cycloalkylcarbamoyl, wie Adamantylcarbamoyl, sein.

Cycloalkylniederalkylcarbamoyl ist beispielsweise N-(C₃-C₆-Cycloalkyl)-C₁-C₄-alkylcarbamoyl, wie N-(Cyclopropylmethyl)carbamoyl, N-(Cyclobutylmethyl)carbamoyl, N-(Cyclopentylmethyl)carbamoyl oder N-(Cyclohexylmethyl)carbamoyl.

Dihydroxyniederalkyl ist beispielsweise β,γ-Dihydroxy-C₃-C₄-alkyl, wie 2, 3-Dihydroxypropyl. Diniederalkylamino ist beispielsweise N,N-Di-C₁-C₇-Alkylamino, vorzugsweise N,N-Di-C₁-C₄-Alkylamino, wie insbesondere Dimethylamino, oder in zweiter Linie Diethylamino, Dipropylamino, Diisopropylamino oder Dibutylamino.

Diniederalkylaminoniederalkylcarbamoyl ist beispielsweise N,N-Di-C₁-C₄-Alkylamino-C₂-C₄-alkylcarbamoyl, wie N-(2-Dimethylaminoethyl)carbamoyl.

Diniederalkylcarbamoyl ist beispielsweise N,N-Di-C₁-C₇-Alkylcarbamoyl, vorzugsweise N,N-Di-C₁-C₄-Alkylcarbamoyl, wie insbesondere Dimethylcarbamoyl, oder in zweiter Linie Diethylcarbamoyl, Dipropylcarbamoyl, Diisopropylcarbamoyl oder Dibutylcarbamoyl.

Diniederalkylphosphono ist beispielsweise Di-C₁-C₇-Alkylphosphono, vorzugsweise Di-C₁-C₄-Alkylphosphono, wie insbesondere Dimethylphosphono, oder in zweiter Linie Diethylphosphono, Dipropylphosphono, Diisopropylphosphono oder Dibutylphosphono.

Halogen ist beispielsweise Halogen der Atomnummer bis und mit 35, wie Chlor oder Fluor, ferner Brom.

Hydroxyniederalkylcarbamoyl ist beispielsweise N-(Hydroxy-C₂-C₄-alkyl)carbamoyl, wie Hydroxymethylcarbamoyl oder 2-Hydroxyethylcarbamoyl.

Niederalkanoyl ist beispielsweise N-C₁-C₇-Alkanoyl, insbesondere N-C₁-C₄-Alkanoyl, wie Formyl, Acetyl, Propionyl, Butyryl oder Isobutyryl, kann aber auch C₅-C₇-Alkanoyl, wie Pivaloyl, sein.

Niederalkanoylamino ist beispielsweise N-C₁-C₇-Alkanoylamino, insbesondere N-C₁-C₄-Alkanoylamino, wie Formylamino, Acetylamino, Propionylamino, Butyrylamino oder Isobutyrylamino, kann aber auch C₅-C₇-Alkanoylamino, wie Pivaloylamino, sein.

N-Niederalkanoyl-N-niederalkyl-amino ist beispielsweise N-C₁-C₇-Alkanoyl-N-C₁-C₄-alkylamino, insbesondere N-C₁-C₄-Alkanoyl-N-C₁-C₄-alkylamino, wie N-Formyl-N-methyl-amino, N-Acetyl-N-methyl-amino, N-Acetyl-N-ethyl-amino, N-Ethyl-N-propionyl-amino, N-Methyl-N-propionyl-amino, N-Butyryl-N-methyl-amino oder N-lsobutyryl-N-methyl-amino.

Niederalkanoyloxy ist beispielsweise N-C₁-C₇-Alkanoyloxy, insbesondere N-C₁-C₄-Alkanoyloxy, wie Formyloxy, Acetoxy, Propionyloxy, Butyryloxy oder Isobutyryloxy, kann aber auch C₅-C₇-Alkanoyloxy, wie Pivaloyloxy, sein.

Niederalkanoyloxyniederalkoxycarbonyl ist beispielsweise N-C₁-C₄-Alkanoyloxy-C₁-C₄-alkoxycarbonyl, wie Acetoxymethoxycarbonyl, Propionyloxymethoxycarbonyl, Butyryloxymethoxycarbonyl oder Pivaloyloxymethoxycarbonyl.

Niederalkoxycarbonylniederalkoxycarbonyl ist beispielsweise C₁-C₄-Alkoxycarbonyl-C₁-C₄-alkoxycarbonyl, wie Methoxycarbonylmethoxycarbonyl, Ethoxycarbonylmethoxycarbonyl oder 2-Methoxycarbonylethoxycarbonyl.

Niederalkenyl ist beispielsweise C₃-C₄-Alkenyl, wie Allyl oder Methallyl.

Niederalkenyloxy ist beispielsweise C₃-C₄-Alkenyloxy, wie Allyloxy oder Methallyloxy.

N-Niederalkenyloxycarbamoyl ist beispielsweise N-C₂-C₄-alkenyloxycarbamoyl, wie N-Vinyloxycarbamoyl, N-Allyloxycarbamoyl oder N-Methallyloxycarbamoyl.

Niederalkinyl ist beispielsweise C₃-C₄-Alkinyl, wie Propargyl.

Niederalkinyloxy ist beispielsweise C₃-C₄-Alkinyloxy, wie Propargyloxy.

Niederalkoxy ist beispielsweise C₁-C₇-Alkoxy, vorzugsweise C₁-C₄-Alkoxy, wie Methoxy, Ethoxy, Propyloxy, Isopropyloxy oder Butyloxy, kann aber auch Isobutyloxy, Sekundärbutyloxy, Tertiärbutyloxy oder eine C₅-C₇-Alkoxy-, wie Pentyloxy-, Hexyloxy- oder Heptyloxygruppe sein.

N-Niederalkoxycarbamoyl ist beispielsweise N-C₁-C₄-Alkoxycarbamoyl, wie Methoxycarbamoyl, Ethoxycarbamoyl, Propyloxycarbamoyl, Isopropyloxycarbamoyl, Butyloxycarbamoyl oder insbesondere Tertiärbutyloxycarbamoyl.

Niederalkoxycarbonyl ist beispielsweise C₁-C₇-Alkoxycarbonyl, vorzugsweise C₁-C₄-Alkoxycarbonyl, wie Methoxycarbonyl, Ethoxycarbonyl, Propyloxycarbonyl, Isopropyloxycarbonyl oder Butyloxycarbonyl , kann aber auch Isobutyloxycarbonyl, Sekundärbutyloxycarbonyl, Tertiärbutyloxycarbonyl oder eine Pentyloxycarbonyl-, Hexyloxycarbonyl- oder Heptyloxycarbonylgruppe sein.

Niederalkyl ist beispielsweise C₁-C₇-Alkyl, vorzugsweise C₁-C₄-Alkyl, wie insbesondere Methyl oder in zweiter Linie Ethyl, Propyl, Isopropyl oder Butyl, kann aber auch Isobutyl, Sekundärbutyl, Tertiärbutyl oder eine C₅-C₇-Alkyl-, wie Pentyl-, Hexyl- oder Heptylgruppe sein.

Niederalkylamino ist beispielsweise N-C₁-C₇-Alkylamino, vorzugsweise N-C₁-C₄-Alkylamino, wie insbesondere Methylamino oder in zweiter Linie Ethylamino, Propylamino, Isopropylamino oder Butylamino, kann aber auch Isobutylamino, Sekundärbutylamino, Tertiärbutylamino oder eine C₅-C₇-Alkylamino-, wie Pentylamino-, Hexylamino-oder Heptylaminogruppe sein.

Niederalkylcarbamoyl ist beispielsweise N-C₁-C₇-Alkylcarbamoyl, vorzugsweise N-C₁-C₄-Alkylcarbamoyl, wie insbesondere Methylcarbamoyl oder in zweiter Linie Ethylcarbamoyl, Propylcarbamoyl, Isopropylcarbamoyl oder Butylcarbamoyl, kann aber auch Isobutylcarbamoyl, Sekundärbutylcarbamoyl, Tertiärbutylcarbamoyl oder eine C₅-C₇-Alkylcarbamoyl-, wie Pentylcarbamoyl-, Hexylcarbamoyl- oder Heptylcarbamoylgruppe sein.

Niederalkylen kann geradkettig oder verzweigt sowie in beliebiger Stellung gebunden sein und ist beispielsweise geradkettiges oder verzweigtes C₁-C₇-Alkylen, vorzugsweise C₁-C₄-Alkylen, wie Methylen, 1,2-Ethylen, 1,3- oder 1,2-Propylen, 1,4-, 1,3- oder 2,3-Butylen oder in zweiter Linie 1,5-, 1,4- oder 2,5-Pentylen.

Niederalkyliden kann geradkettig oder verzweigt sowie geminal in beliebiger Stellung gebunden sein und ist beispielsweise geradkettiges oder verzweigtes C₁-C₇-Alkylen, vorzugsweise C₁-C₄-Alkylen, wie Methylen, 1,1-Ethyliden, 1,1-oder oder 2,2-Propyliden, 1,1-oder oder 2,2-Butyliden oder in zweiter Linie 1,1-oder 2,2-Pentyliden.

N-Niederalkyl-N-phenyl-carbamoyl ist beispielsweise N-C₁-C₄-Alkyl-N-phenyl-carbamoyl, wie insbesondere N-Methyl-N-phenyl-carbamoyl oder in zweiter Linie N-Ethyl-N-phenyl-carbamoyl, N-Propyl-N-phenyl-carbamoyl, N-Isopropyl-N-phenyl-carbamoyl oder N-Butyl-N-phenyl-carbamoyl, kann aber auch N-Isobutyl-N-phenyl-carbamoyl, N-Sekundärbutyl-N-phenyl-carbamoyl oder N-Tertiärbutyl-N-phenyl-carbamoyl sein.

Niederalkylphosphono ist beispielsweise C₁-C₇-Alkylphosphono, vorzugsweise C₁-C₄-Alkylphosphono, wie insbesondere Methylphosphono oder in zweiter Linie Ethylphosphono, Propylphosphono, Isopropylphosphono oder Butylphosphono, kann aber auch Isobutylphosphono, Sekundärbutylphosphono, Tertiärbutylphosphono oder eine C₅-C₇-Alkylphosphono-, wie Pentylphosphono-, Hexylphosphono- oder Heptylphosphonogruppe sein.

Oxaniederalkylenaminoniederalkylcarbamoyl ist beispielsweise N-(Morpholino-C₂-C₄-alkyl)carbamoyl, wie insbesondere N-(2-Morpholinoethyl)ethylcarbamoyl.

2-Oxoimidazolidin-1-ylniederalkylencarbamoyl ist beispielsweise N-(2-Oxoimidazolidin-1-yl-C₄-C₅-alkylen)carbamoyl, wie N-[4-(2-Oxoimidazolidin-1-yl)piperidinocarbonyl.

2-Oxoimidazolidin-1-ylniederalkylcarbamoyl ist beispielsweise N-(2-Oxoimidazolidin-1-yl-C₂-C₅-alkyl)carbamoyl, wie N-(2-Oxoimidazolidin-1-yl)ethylcarbamoyl.

N-Phenylniederalkoxycarbamoyl ist beispielsweise unsubstituiertes oder durch C₁-C₄-Alkyl, C₁-C₄-Alkoxy, Hydroxy, Halogen und/oder Trifluormethyl substituiertes Phenyl-C₁-C₄-alkoxycarbamoyl, wie Benzyloxycarbamoyl oder 1-Phenylethoxycarbamoyl.

N-Phenylniederalkenyloxycarbamoyl ist beispielsweise N-Phenyl-C₂-C₄-alkenyloxycarbamoyl, wie N-Phenylvinyloxycarbamoyl oder N-(3-Phenylprop-2-enyloxy)carbamoyl.

Phenylniederalkoxycarbonyl ist beispielsweise unsubstituiertes oder durch C₁-C₄-Alkyl, C₁-C₄-Alkoxy, Hydroxy, Halogen und/oder Trifluormethyl substituiertes Phenyl-C₁-C₄-alkoxycarbonyl, wie Benzyloxycarbonyl oder 1-Phenylethoxycarbonyl.
Phenylniederalkylcarbamoyl ist beispielsweise unsubstituiertes oder durch C₁-C₄-Alkyl, C₁-C₄-Alkoxy, Hydroxy, Halogen und/oder Trifluormethyl substituiertes Phenyl-C₁-C₄-alkylcarbamoyl, wie Benzylcarbamoyl oder 2-Phenylethylcarbamoyl.

Triniederalkylphosphono ist beispielsweise Tri-C₁-C₇-Alkylphosphono, vorzugsweise Tri-C₁-C₄-Alkylphosphono, wie insbesondere Trimethylphosphono, oder in zweiter Linie Triethylphosphono, Tripropylphosphono, Triisopropylphosphono oder Tributylphosphono.

Verbindungen der Formel 1, die saure Gruppen aufweisen, beispielsweise solche in denen mindestens einer der Reste R₁, R₂, R₄ und R₅ Carboxy, Phosphono oder Tetrazolyl bedeutet oder aufweist, können Salze mit Basen oder innere Salze bilden. Verbindungen der Formel I können ferner Säureadditionssalze bilden.

Salze von Verbindungen der Formel I mit Basen sind beispielsweise deren Salze mit pharmazeutisch verwendbaren Basen, wie nicht-toxische, von Metallen der Gruppen Ia, Ib, IIa und IIb abgeleitete Metallsalze, z.B. Alkalimetall-, insbesondere Natrium- oder Kaliumsalze, Erdalkalimetall-, insbesondere Calcium- oder Magnesiumsalze, ebenso Ammoniumsalze mit Ammoniak oder organischen Aminen oder quaternären Ammoniumbasen, wie gegebenenfalls C-hydroxylierten aliphatischen Aminen, insbesondere Mono-, Di- oder Triniederalkylaminen, z.B. Methyl-, Ethyl- oder Diethylamin, Mono-, Di- oder Tri-(hydroxyniederalkyl)aminen, wie Ethanol-, Diethanol- oder Triethanolamin, Tris-(hydroxymethyl)methylamin oder 2-Hydroxytertiärbutylamin, oder N-(Hydroxyniederalkyl)-N,N-diniederalkyl-aminen bzw. N-(Polyhydroxyniederalkyl)-N-niederalkylaminen, wie 2-(Dimethylamino)ethanol oder D-Glucamin oder Cholin, oder quaternären aliphatischen Ammoniumhydroxiden, z.B. Tetrabutylammoniumhydroxid.

Säureadditionssalze von Verbindungen der Formel I sind beispielsweise deren pharmazeutisch verwendbare Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, oder Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate).

Umfaßt sind sowohl vollständige als auch partielle Salze, d.h. Salze mit 1, 2 oder 3, vorzugsweise 2, Äquivalenten Base pro Mol Säure der Formel I bzw. Salze mit 1, 2 oder 3 Äquivalenten, vorzugsweise 1 Äquivalent, Säure pro Mol Base der Formel I.

Zur Isolierung oder Reinigung können auch pharmazeutisch ungeeignete Salze Verwendung finden. Zur therapeutischen Anwendung gelangen nur die pharmazeutisch verwendbaren, nicht-toxischen Salze, die deshalb bevorzugt sind.

Die Verbindungen der Formel I weisen wertvolle pharmakologische Eigenschaften auf. Sie besitzen eine selektive nicht-kompetitive antagonistische Wirkung gegenüber N-Methyl-D-asparaginsäure-sensitiven (NMDA-sensitiven) excitatorischen Aminosäurerezeptoren von Warmblütern. Insbesondere vermögen sie an Strychnin-insensitive Glycinbindungsstellen des NMDA-Rezeptors zu binden. Das Bindungsvermögen der erfindungsgemäß bereitgestellten Verbindungen und ihrer Salze an Strychnin-insensitive Glycinbindungsstellen des NMDA-Rezeptorkomplexes kann *in vitro* beispielsweise in der Versuchsanordnung nach Baron et al., Eur. J. Pharmacol., Molec. Pharmacol. Section 206, Seiten 149-154 (1991) und Canton et al., J. Pharm. Pharmacol. 44, Seiten 812-816 (1992) an Rattenkortex- und Rattenhippocampusmembranen ermittelt werden. Dabei wird bestimmt, in welchem Ausmaß [³H]-5,7-Dichlorkynurensäure (³H-DCKA) verdrängt wird, wobei die prozentuale Hemmung und gegebenenfalls bei mehreren Konzentrationen die für eine 50 %-iger Verdrängung erforderliche Konzentration (IC₅₀) bestimmt wird. Die für eine 50 %-ige Verdrängung erforderliche Konzentration (IC₅₀) liegt im Nano- und unteren Millimolbereich, d.h. bei Konzentrationen von etwa 0.01 bis 10 µMol.

Auf Grund dieser Eigenschaften sind die Verbindungen der Formel I und ihre pharmazeutisch verwendbaren Salze vorzüglich geeignet zur prophylaktischen und therapeutischen Behandlung von pathologischen Zuständen, die auf Blockierung von Glycinbindungsstellen ansprechen, beispielsweise von neurodegenerativen Erkrankungen, wie solcher im Gefolge von Schlaganfall, Hypoglykämie, Anoxie oder Hirnlähmungserscheinungen, von ischämischen Erkrankungen, wie der cerebralen Ischämie, der cerebralen Ischämie bei Herzchirurgie oder Herzstillstand, perinataler Aphyxie, epilepstischer Anfälle, chorea Huntington, Alzheimer'scher und Parkinson'scher Erkrankung, amyotroper Lateralsklerose, Rückermark- und Hirntrauma sowie Vergiftungserscheinungen durch Neurotoxine oder Suchtmittelmissbrauch, und von ischämischen Erkrankungen des Auges, von Gefäß- und Muskelspasmen, wie von Migräne oder von lokaler oder generaler Spastizität, von Konvulsionen, wie der Epilepsie, und von Angst- und Schmerzzuständen, wie Trigeminusneuralgien.

Die antikonvulsiven Eigenschaften der erfindungsgemäßen Verbindungen können in vivo beispielsweise an der Maus anhand ihrer ausgeprägten Schutzwirkung gegenüber durch Elektroschock oder Metrazol ausgelösten Konvulsionen bestimmt werden, wobei man z.B. das etablierte Elektroschck-Mausmodell bzw. das Mausmodell für Metrazol-induzierte Konvulsionen gemäß Schmutz et al., Naunyn-Schmiedeberg's Arch. Pharmacol. 342, 61-66 (1990) heranziehen kann.

Die Erfindung betrifft beispielsweise Verbindungen der Formel I, worin
A₁ Methylen oder eine Gruppe der Formel >CH-A₄-R₄ (Ia) darstellt,
A₂ Niederalkyliden oder eine Gruppe der Formel >C=O (Ib) ist,
A₃ eine Gruppe der Formel >N-A₆-R₅ (Id) bedeutet,
A₄ für Niederalkylen steht,
A₆ Niederalkylen oder eine direkte Bindung darstellt,
n für 0 oder 1 steht,
R₁ und R₂ unabhängig voneinander Wasserstoff, gegebenenfalls durch Niederalkyl und/oder Niederalkanoyl substituiertes Amino, gegebenenfalls verethertes Hydroxy, gegebenenfalls halogeniertes Niederalkyl oder Halogen bedeuten,
R₃ Wasserstoff oder Hydroxy bedeutet,
R₄ gegebenenfalls verestertes oder amidiertes Carboxy darstellt und
R₅ Wasserstoff, Aryl, Niederalkanoyl, gegebenenfalls verestertes oder amidiertes Carboxy, Cyano oder gegebenenfalls verethertes Hydroxy bedeutet,
und ihre Salze, die neuen Verbindungen enthaltende pharmazeutische Präparate und ihre Verwendung als Arzneimittelwirkstoffe.

Die Erfindung betrifft in erster Linie Verbindungen der Formel I, worin
A₁ Niederalkyliden oder eine Gruppe der Formel >CH-A₄-R₄ (Ia) darstellt,
A₂ Niederalkyliden oder eine Gruppe der Formeln >CH-A₄-R₄ (Ia) oder >C=O (Ib) oder >CH(OH)-A₅-R₅ (Ic) bedeutet,
A₃ eine Gruppe der Formel >N-A₆-R₅ (Id) darstellt,
A₄ für Niederalkylen steht,
A₅ und A₆ unabhängig voneinander Niederalkylen oder eine direkte Bindung darstellen,
n für 0 oder 1 steht,
R₁ und R₂ unabhängig voneinander Wasserstoff, Nitro, Niederalkanoyl, Amino, Niederalkylamino, Niederalkanoylamino, Diniederalkylamino oder N-Niederalkyl-N-niederalkanoylamino, Carboxy, Niederalkoxycarbonyl, Carbamoyl, Cyano, Hydroxy, Niederalkoxy, Niederalkenyloxy, Niederalkinyloxy, Niederalkyl, Polyhalogenniederalkyl oder Halogen bedeuten, R₃ Wasserstoff oder Hydroxy bedeutet,
R₄ Carboxy, Niederalkoxycarbonyl, Carboxyniederalkoxycarbonyl, Niederalkoxycarbonylniederalkoxycarbonyl, Niederalkanoyloxyniederalkoxycarbonyl, unsubstituiertes oder durch Niederalkyl, Niederalkoxy, Hydroxy, Halogen und/oder Trifluormethyl substituiertes Phenylniederalkoxycarbonyl, Carbamoyl, Niederalkylcarbamoyl, Hydroxyniederalkylcarbamoyl, Diniederalkylcarbamoyl, Diniederalkylaminoniederalkylcarbamoyl, Aminoniederalkylaminoniederalkylcarbamoyl, 2-Oxoimidazolidin-1-ylniederalkylcarbamoyl, Aminoniederalkylaminoniederalkylencarbamoyl, 2-Oxoimidazolidin-1-ylniederalkylencarbamoyl, Oxaniederalkylenaminoniederalkylcarbamoyl, unsubstituiertes oder durch Carboxy oder Niederalkoxycarbonyl substituiertes Cycloalkylcarbamoyl, Cycloalkylniederalkylcarbamoyl, unsubstituiertes oder durch Niederalkyl, Niederalkoxy, Hydroxy, Halogen und/oder Trifluormethyl substituiertes Phenylniederalkylcarbamyol, unsubstituiertes oder durch Niederalkyl, Niederalkoxy, Hydroxy, Halogen, Nitro, Carboxy, Niederalkoxycarbonyl, Phenyl, Phenyloxy und/oder Trifluormethyl substituiertes N-Phenyl- oder N-Niederalkyl-N-phenylcarbamoyl, N-Hydroxycarbamoyl, N-Niederalkoxycarbamoyl, N-Niederalkenyloxycarbamoyl, unsubstituiertes oder durch Niederalkyl, Niederalkoxy, Hydroxy, Halogen und/oder Trifluormethyl substituiertes N-Phenylniederalkoxy- oder N-Phenylniederalkenyloxycarbamoyl, Phophono, Niederalkylphosphono, Diniederalkylphosphono, Triniederalkylphosphono oder 5-Tetrazolyl darstellt und
R₅ Wasserstoff, Niederalkyl, Niederalkenyl, Niederalkinyl, unsubstituiertes oder durch Niederalkyl, Niederalkoxy, Hydroxy, Halogen und/oder Trifluormethyl substituiertes Phenyl, Niederalkanoyl, Carboxy, Niederalkoxycarbonyl unsubstituiertes oder durch Niederalkyl, Niederalkoxy, Hydroxy, Halogen und/oder Trifluormethyl substituiertes Phenylniederalkoxycarbonyl, Carbamoyl, Niederalkylcarbamoyl, Diniederalkylcarbamoyl, unsubstituiertes oder durch Niederalkyl, Niederalkoxy, Hydroxy, Halogen, Nitro, Carboxy, Niederalkoxycarbonyl, Phenyl, Phenyloxy und/oder Trifluormethyl substituiertes N-Phenyl- oder N-Niederalkyl-N-phenylcarbamoyl, Cyano, Tetrazolyl, Hydroxy, Niederalkanoyloxy, Niederalkoxy, Niederalkenyloxy, Niederalkinyloxy oder Dihydroxyniederalkyl bedeutet, und ihre Salze.

Die Erfindung betrifft vor allem Verbindungen der Formel I, worin
A₁ C₁-C₄-Alkyliden, wie Methylen, oder eine Gruppe der Formel >CH-A₄-R₄ (Ia) darstellt,
A₂ C₁-C₄-Alkyliden, wie Methylen, oder eine Gruppe der Formeln >CH-A₄-R₄ (Ia) oder >C=O (Ib) bedeutet,
A₃ eine Gruppe der Formel >N-A₆-R₅ (Id) darstellt,
A₄ für C₁-C₄-Alkylen steht,
A₅ und A₆ unabhängig voneinander C₁-C₄-Alkylen, wie Methylen, oder eine direkte Bindung darstellen,
n für 0 steht,
R₁ und R₂ unabhängig voneinander Wasserstoff, Nitro, C₁-C₇-Alkanoyl, wie Propionyl, Amino, C₁-C₇-Alkylamino, wie Methylamino, C₁-C₇-Alkanoylamino, wie Acetamino, Di-C₁-C₄-alkylamino, wie Dimethylamino, oder N-C₁-C₄-Alkyl-N-C₁-C₇-alkanoyl-amino, wie N-Acetyl-N-methyl-amino, Hydroxy, Carboxy, C₁-C₄-Alkoxycarbonyl, wie Methoxy- oder Ethoxycarbonyl, Carbamoyl, Cyano, C₁-C₄-Alkoxy, wie Methoxy, C₂-C₄-Alkenyloxy, wie Allyloxy, C₂-C₄-Alkinyloxy, wie Propargyloxy, C₁-C₄-Alkyl, wie Methyl oder Ethyl, Trifluormethyl oder Halogen, wie Fluor, Chlor oder Brom, bedeuten,
R₃ Wasserstoff oder Hydroxy bedeutet,
R₄ Carboxy, C₁-C₄-Alkoxycarbonyl, wie Methoxy- oder Ethoxycarbonyl, Carboxy-C₁-C₄-alkoxycarbonyl, wie Carboxymethoxycarbonyl, C₁-C₄-Alkoxycarbonyl-C₁-C₄-alkoxycarbonyl, wie Methoxy- oder Ethoxycarbonylmethoxycarbonyl, C₁-C₄-Alkanoyloxy-C₁-C₄-alkoxycarbonyl, wie 2-Acetoxyethoxycarbonyl, unsubstituiertes oder durch C₁-C₄-Alkyl, wie Methyl, C₁-C₄-Alkoxy, wie Methoxy, Hydroxy, Halogen, wie Chlor, und/oder Trifluormethyl substituiertes Phenyl-C₁-C₄-alkoxycarbonyl, wie Benzyloxycarbonyl, Carbamoyl, C₁-C₇-Alkylcarbamoyl, wie Methyl- oder Butylcarbamoyl, Hydroxy-C₁-C₄-alkylcarbamoyl, wie 2-Hydroxyethylcarbamoyl, Di-C₁-C₄-alkylcarbamoyl, wie Dimethyl- oder Dibutylcarbamoyl, Di-C₁-C₄-alkylamino-C₁-C₄-alkylcarbamoyl, wie 2-(2-Dimethylaminoethyl)ethylcarbamoyl, Amino-C₁-C₄-alkylamino-C₁-C₄-alkylcarbamoyl, wie 2-(2-Aminoethyl)ethylcarbamoyl, 2-Oxoimidazolidin-1-yl-C₁-C₄-alkylcarbamoyl, wie 2-(2-Oxoimidazolidin-1-yl-ethylcarbamoyl, Amino-C₁-C₄-alkylamino-C₁-C₄-alkylencarbamoyl, wie 4-(2-Aminoethyl)piperidinocarboyl, 2-Oxoimidazolidin-1-ylniederalkylencarbamoyl, wie 4-(2-Oxoimidazolidin-1-yl)piperidinocarbonyl, Oxa-C₁-C₄-alkylenamino-C₁-C₄-alkylcarbamoyl, wie 2-Morpholinoethylcarbamoyl, unsubstituiertes oder durch Carboxy oder C₁-C₄-Alkoxycarbonyl substituiertes Cycloalkylcarbamoyl, wie Cyclopropylcarbamoyl, N-(1-Methoxycarbonylcyclopropyl)carbamoyl, Adamantylcarbamoyl, Cycloalkyl-C₁-C₄-alkylcarbamoyl, wie Cyclopropylmethylcarbamoyl, unsubstituiertes oder durch C₁-C₄-Alkyl, wie Methyl, C₁-C₄-Alkoxy, wie Methoxy, Hydroxy, Halogen, wie Chlor, und/oder Trifluormethyl substituiertes Phenyl-C₁-C₄-alkylcarbamoyl, unsubstituiertes oder durch C₁-C₄-Alkyl, wie Methyl, C₁-C₄-Alkoxy, wie Methoxy, Hydroxy, Halogen, wie Chlor oder Fluor, Nitro, Carboxy, C₁-C₄-Alkoxycarbonyl, wie Methoxycarbonyl, Phenyl, Phenyloxy und/oder Trifluormethyl substituiertes N-Phenyl- oder N-C₁-C₄-Alkyl-N-phenyl-carbamoyl, wie N-Methyl-N-phenyl-carbamoyl, N-Hydroxycarbamoyl, N-C₁-C₄-Alkoxycarbamoyl, wie N-Tetiärbutyloxycarbamoyl, unsubstituiertes oder durch C₁-C₄-Alkyl, wie Methyl, C₁-C₄-Alkoxy, wie Methoxy, Hydroxy, Halogen, wie Chlor, und/oder Trifluormethyl substituiertes N-Phenyl-C₁-C₄-alkoxycarbamoyl, wie N-Benzyloxycarbamoyl, Phosphono, C₁-C₇-Alkylphosphono, vorzugsweise C₁-C₄-Alkylphosphono, wie Methylphosphono, Ethylphosphono, Propylphosphono, Isopropylphosphono oder Butylphosphono, Isobutylphosphono, Sekundärbutylphosphono oder Tertiärbutylphosphono, Di-C₁-C₇-Alkylphosphono, vorzugsweise Di-C₁-C₄-Alkylphosphono, wie Dimethylphosphono, Diethylphosphono, Dipropylphosphono, Diisopropylphosphono oder Dibutylphosphono, Tri-C₁-C₇-Alkylphosphono, vorzugsweise Tri-C₁-C₄-Alkylphosphono, wie Trimethylphosphono, Triethylphosphono, Tripropylphosphono, Triisopropylphosphono oder Tributylphosphono, oder 5-Tetrazolyl darstellt und
R₅ Wasserstoff, C₁-C₄-Alkyl, wie Methyl, C₂-C₄-Alkenyl, wie Allyl, C₂-C₄-Alkinyl, wie Propargyl, unsubstituiertes oder durch C₁-C₄-Alkyl, wie Methyl, C₁-C₄-Alkoxy, wie Methoxy, Hydroxy, Halogen, wie Chlor, und/oder Trifluormethyl substituiertes Phenyl, C₁-C₄-Alkanoyl, wie Acetyl, Carboxy, C₁-C₄-Alkoxycarbonyl, wie Methoxy- oder Ethoxycarbonyl, Carbamoyl, C₁-C₄-Alkylcarbamoyl, wie Methylcarbamoyl, Di-C₁-C₄-alkylcarbamoyl, wie Dimethylcarbamoyl, unsubstituiertes oder durch C₁-C₄-Alkyl, wie Methyl, C₁-C₄-Alkoxy, Wie Methoxy, Hydroxy, Halogen, wie Fluor oder Chlor, Nitro, Carboxy, C₁-C₄-Alkoxycarbonyl, wie Methoxycarbonyl, Phenyl, Phenyloxy und/oder Trifluormethyl substituiertes N-Phenyl- oder N-C₁-C₄-Alkyl-N-phenyl-carbamoyl, wie N-Methyl-N-phenyl-carbamoyl, Cyano, Tetrazolyl oder Dihydroxy-C₂-C₄-alkyl, wie 2,3-Dihydroxypropyl, bedeutet, und ihre Salze.

Die Erfindung betrifft vor allem beispielsweise Verbindungen der Formel 1, worin
A₁ eine Gruppe der Formel >CH-A₄-R₄ (Ia) darstellt,
A₂ C₁-C₄-Alkyliden, wie Methylen, 1,1-Ethyliden, 1,1-oder 2,2- Propyliden oder 1,1-oder 2,2-Butyliden, oder Carbonyl ist,
A₃ eine Gruppe der Formel >N-A₅-R₅ (Ic) bedeutet,
A₄ für geradkettiges oder verzweigtes C₁-C₄-Alkylen, wie Methylen, 1,1-Ethyliden, 1,1- oder 2,2- Propyliden oder 1,1- oder 2,2-Butyliden, steht,
A₅ geradkettiges oder verzweigtes C₁-C₄-Alkylen, wie Methylen, 1,2-Ethylen, 1,3- oder 1,2-Propylen oder 1,4-, 1,3- oder 2,3-Butylen, oder eine direkte Bindung darstellt,
n für 0 steht,
R₁ und R₂ unabhängig voneinander Wasserstoff, C₁-C₄-Alkoxy, wie Methoxy, C₁-C₄-Alkyl, wie Methyl, Ethyl, Propyl, Isopropyl, Butyl, Isobutyl, Sekundärbutyl oder Tertiärbutyl, Trifluormethyl oder Halogen der Atomnummer bis und mit 35, wie Chlor oder Fluor, ferner Brom, bedeuten,
R₃ Wasserstoff bedeutet,
R₄ Carboxy, C₁-C₄-Alkoxycarbonyl, wie Methoxycarbonyl, Ethoxycarbonyl, Propyloxycarbonyl, Isopropyloxycarbonyl, Butyloxycarbonyl, Isobutyloxycarbonyl, Sekundärbutyloxycarbonyl, oder Tertiärbutyloxycarbonyl, unsubstituiertes oder durch C₁-C₄-Alkyl, wie Methyl, C₁-C₄-Alkoxy, wie Methoxy, Hydroxy, Halogen der Atomnummer bis und mit 35, wie Chlor, und/oder Trifluormethyl substituiertes Phenyl-C₁-C₄-alkoxycarbonyl, wie Benzyloxycarbonyl oder 1-Phenylethoxycarbonyl, Carbamoyl, N-C₁-C₇-Alkylcarbamoyl, vorzugsweise N-C₁-C₄-Alkylcarbamoyl, wie Methylcarbamoyl, Ethylcarbamoyl, Propylcarbamoyl, Isopropylcarbamoyl, Butylcarbamoyl, Isobutylcarbamoyl, Sekundärbutylcarbamoyl oder Tertiärbutylcarbamoyl, unsubstituiertes oder durch C₁-C₄-Alkyl, wie Methyl, C₁-C₄-Alkoxy, wie Methoxy, Hydroxy, Halogen der Atomnummer bis und mit 35, wie Chlor, und/oder Trifluormethyl substituiertes Phenylcarbamoyl, N,N-Di-C₁-C₄-Alkylcarbamoyl, wie Dimethylcarbamoyl, Diethylcarbamoyl, Dipropylcarbamoyl, Diisopropylcarbamoyl oder Dibutylcarbamoyl, darstellt und
R₅ Wasserstoff, Carboxy, C₁-C₄-Alkoxycarbonyl, wie Methoxycarbonyl, Carbamoyl, N-C₁-C₄-Alkylcarbamoyl, wie Methylcarbamoyl, unsubstituiertes oder durch C₁-C₄-Alkyl, wie Methyl, C₁-C₄-Alkoxy, wie Methoxy, Hydroxy, Halogen der Atomnummer bis und mit 35, wie Chlor, und/oder Trifluormethyl substituiertes Phenylcarbamoyl, N,N-Di-C₁-C₄-Alkylcarbamoyl, wie insbesondere Dimethylcarbamoyl, Cyano, Hydroxy, C₁-C₄-Alkoxy, wie Methoxy, Ethoxy, Propyloxy, Isopropyloxy, Butyloxy, Isobutyloxy, Sekundärbutyloxy oder Tertiärbutyloxy, C₃-C₄-Alkenyloxy, wie Allyloxy oder Methallyloxy, oder C₃-C₄-Alkinyloxy, wie Propargyloxy, bedeutet,
und ihre Salze.

Die Erfindung betrifft in allererster Linie Verbindungen der Formel I, worin
A₁ eine Gruppe der Formel >CH-A₄-R₄ (Ia) darstellt,
A₂ Methylen ist,
A₃ eine Gruppe der Formel >N-A₅-R₅ (Ic) bedeutet,
A₄ für Methylen steht,
n für 0 steht,
A₅ eine direkte Bindung darstellt,
einer der Reste R₁ und R₂ C₁-C₄-Alkyl, wie Methyl, Halogen der Atomnummer bis und mit 35, wie Chlor, Fluor oder Brom, oder Nitro und der andere Wasserstoff oder C₁-C₄-Alkyl, wie Methyl, Halogen der Atomnummer bis und mit 35, wie Chlor, Fluor oder Brom, bedeutet, R₃ Wasserstoff ist,
R₄ Carboxy, Phosphono, C₁-C₄-Alkoxycarbonyl, wie Methoxycarbonyl oder Ethoxycarbonyl, N-Hydroxycarbamoyl, N-C₁-C₄-Alkylcarbamoyl, wie Butyl- oder Tertiärbutylcarbamoyl, N,N-Di-C₁-C₄-alkylcarbamoyl, wie Dibutylcarbamoyl, N-(Carboxy-C₁-C₄-alkyl)carbamoyl, wie Carboxymethylcarbamoyl, N-(C₁-C₄-Alkoxycarbonyl-C₁-C₄-alkyl)carbamoyl, wie N-Methoxycarbonylmethylcarbamoyl oder N-Ethoxycarbonylmethylcarbamoyl, N-(Hydroxy-C₂-C₄-alkyl)carbamoyl, wie 2-Hydroxyethylcarbamoyl, N-C₁-C₄-Alkoxycarbamoyl, wie N-Tertiärbutyloxycarbamoyl, oder eine unsubstituierte oder durch C₁-C₄-Alkyl, wie Methyl, C₁-C₄-Alkoxy, wie Methoxy, Halogen der Atomnummer bis und mit 35, wie Chlor, Trifluormethyl, Nitro, Carboxy und/oder C₁-C₄-Alkoxycarbonyl, wie Methoxycarbonyl oder Ethoxycarbonyl substituierte N-Phenylcarbamoyl-, N-(Phenyl-C₁-C₄-alkyl)carbamoyl-, wie Benzylcarbamoyl- oder 2-Phenylethylcarbamoyl-, oder N-(Phenyl-C₁-C₄-alkoxy)carbamoyl-, wie N-Benzyloxycarbamoylgruppe bedeutet und
R₅ Wasserstoff bedeutet, vorzugsweise solche der Formel I' 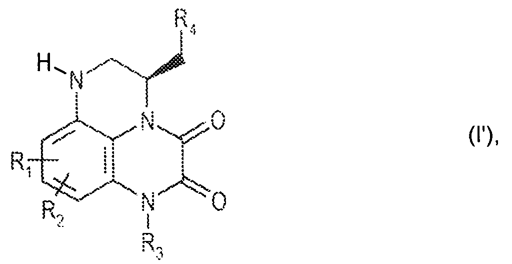
worin
einer der Reste R₁ und R₂ C₁-C₄-Alkyl, wie Methyl, Halogen der Atomnummer bis und mit 35, wie Chlor, Fluor oder Brom, oder Nitro und der andere Wasserstoff oder C₁-C₄-Alkyl, wie Methyl, Halogen der Atomnummer bis und mit 35, wie Chlor, Fluor oder Brom, bedeutet,
R₃ Wasserstoff ist und
R₄ Carboxy, C₁-C₄-Alkoxycarbonyl, wie Methoxycarbonyl oder Ethoxycarbonyl, N-Hydroxycarbamoyl, N-C₁-C₄-Alkylcarbamoyl, wie Butyl- oder Tertiärbutylcarbamoyl, N,N-Di-C₁-C₄-alkylcarbamoyl, wie Dibutylcarbamoyl, N-(Carboxy-C₁-C₄-alkyl)carbamoyl, wie Carboxymethylcarbamoyl, N-(C₁-C₄-Alkoxycarbonyl-C₁-C₄-alkyl)carbamoyl, wie N-Methoxycarbonylmethylcarbamoyl oder N-Ethoxycarbonylmethylcarbamoyl, N-(Hydroxy-C₂-C₄-alkyl)carbamoyl, wie 2-Hydroxyethylcarbamoyl, N-C₁-C₄-Alkoxycarbamoyl, wie N-Tertiärbutyloxycarbamoyl, oder eine unsubstituierte oder durch C₁-C₄-Alkyl, wie Methyl, C₁-C₄-Alkoxy, wie Methoxy, Halogen der Atomnummer bis und mit 35, wie Chlor, Trifluormethyl, Nitro, Carboxy und/oder C₁-C₄-Alkoxycarbonyl, wie Methoxycarbonyl oder Ethoxycarbonyl substituierte N-Phenylcarbamoyl-, N-(Phenyl-C₁-C₄-alkyl)carbamoyl-, wie Benzylcarbamoyl- oder 2-Phenylethylcarbamoyl-, oder N-(Phenyl-C₁-C₄-alkoxy)carbamoyl-, wie N-Benzyloxycarbamoylgruppe bedeutet,
und ihre Salze.

Die Erfindung betrifft in allererster Linie beispielsweise Verbindungen der Formeln I bzw. I', worin
A₁ eine Gruppe der Formel >CH-A₄-R₄ (Ia) darstellt,
A₂ Methylen ist,
A₃ eine Gruppe der Formel >N-A₆-R₅ (Ic) bedeutet,
A₄ für Methylen steht,
n für 0 steht,
A₆ direkte Bindung darstellt,
R₁ und R₂ unabhängig voneinander Wasserstoff oder Halogen der Atomnummer bis und mit
35, wie Chlor, bedeuten,
R₃ Wasserstoff ist,
R₄ Carboxy, C₁-C₄-Alkoxycarbonyl, wie Methoxycarbonyl, Ethoxycarbonyl, Propyloxycarbonyl, Isopropyloxycarbonyl, Butyloxycarbonyl, Isobutyloxycarbonyl, Sekundärbutyloxycarbonyl oder Tertiärbutyloxycarbonyl, oder eine unsubstituierte oder durch C₁-C₄-Alkyl, wie Methyl, C₁-C₄-Alkoxy, wie Methoxy, Hydroxy, Halogen der Atomnummer bis und mit 35, wie Chlor, und/oder Trifluormethyl substituierte N-Phenylcarbamoylgruppe bedeutet und R₅ Wasserstoff bedeutet,
und ihre Salze.

Die Erfindung betrifft namentlich die in den Beispielen genannten Verbindungen der Formel I und ihre Salze, insbesondere ihre pharmazeutisch verwendbaren Salze.

Das Verfahren zur Herstellung der neuen Verbindungen der Formel I beruht auf an sich bekannten Methoden und ist beispielsweise dadurch gekennzeichnet, daß man
eine Verbindung der Formel II 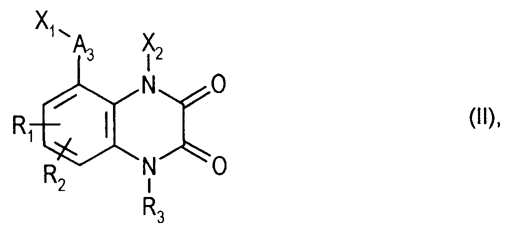
worin
X₁ eine Gruppe der Formel -A₂- (CH₂)ₙ-A₁-Y₁ (IIa) oder -A₂(CH₂)ₙ-CH=A₄-R₄ (IIb) darstellt, wobei
Y₁ eine nukleofuge Abgangsgruppe bedeutet,
intramolekular cyclisiert und gewünschtenfalls eine erhaltene Verbindung in eine andere Verbindung der Formel I überführt, ein verfahrensgemäß erhältliches Isomerengemisch in die Komponenten auftrennt und das jeweils bevorzugte Isomere abtrennt und/oder eine verfahrensgemäß erhältliche freie Verbindung in ein Salz oder eine verfahrensgemäß erhältliches Salz in die entsprechende freie Verbindung überführt.

Nukleophile Abgangsgruppen sind in diesem Zusammenhang beispielsweise reaktionsfähige veresterte Hydroxygruppen, wie mit einer Mineralsäure oder Sulfonsäure veresterte Hydroxygruppen, insbesondere Halogenatome, beispielsweise Chlor, Brom oder Jod, oder mit einer aliphatischen oder einer gegebenenfalls substituierten aromatischen Sulfonsäure veresterte Hydroxygruppen, beispielsweise Niederalkansulfonyloxy, wie Methansulfonyloxy, oder gegebenenfalls substituiertes Benzolsulfonyloxy, wie Benzolsulfonyloxy, Brombenzolsulfonyloxy oder Toluolsulfonyloxy.

Die intramolekulare Cyclisierung von Verbindungen der Formel II erfolgt in üblicher Weise, vorzugsweise in einem inerten organischen Lösungsmittel, wie Tetrahydrofuran, Dioxan oder Dimethylformamid, erforderlichenfalls in Gegenwart eines basischen Kondensationsmittels, wie eines tertiären aliphatischen Amins, wie Triethylamin, oder einer tertiären aromatischen Stickstoffbase, wie Pyridin, oder einer Metallbase, wie einem Alkalimetallhydroxid, Alkalimetallcarbonat oder Alkalimetallamid, z.B. Natrium- oder Kaliumhydroxid, Natrium- oder Kaliumcarbonat oder Natrium- oder Kaliumamid, vorteilhaft unter Erwärmen, z.B. im Temperaturbereich von etwa 25° bis 120°, vorzugsweise zwischen 50° und 120°.

Ausgangsstoffe der Formel II werden vorzugsweise in situ gebildet und ohne Isolierung erfindungsgemäß cyclisiert, beispielsweise indem man eine Verbindungen der Formel III 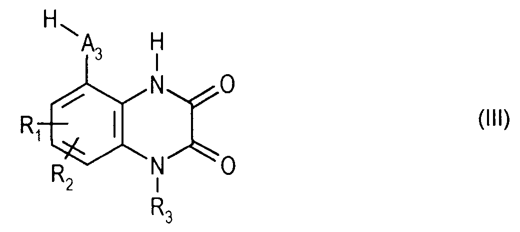
mit einer Verbindung der Formeln Y₂-A₂-(CH₂)ₙ-A₁-Y₁ (IV) bzw. Y₂A₂-(CH₂)ₙ=A₄R₄ (V), worin Y₁ und Y₂ gleiche oder verschiedene nukleofuge Abgangsgruppen bedeuten, mit einander umsetzt.

Verfahrensgemäss erhältliche Verbindungen können in üblicher Weise in andere Verbindungen der Formel I überführt werden.

So kann man Verbindungen der Formel I, worin R₄ und gegebenenfalls R₅ für Carboxy steht, zu der entsprechenden Verbindung der Formel I verestern, in der R₄ und gegebenenfalls R₅ für verestertes Carboxy steht. Ebenso kann man Verbindungen der Formel 1, worin R₄ und gegebenenfalls R₅ für gegebenenfalls verestertes Carboxy steht, zu der entsprechenden Verbindung der Formel I amidieren, in der R₄ und gegebenenfalls R₅ für amidiertes Carboxy steht. Umgekehrt kann man Verbindungen der Formel I, worin R₅ für Cyano oder R₄ und gegebenenfalls R₅ für verestertes oder amidiertes Carboxy steht, zu der entsprechenden Verbindung der Formel I hydrolysieren, in der R₄ und gegebenenfalls R₅ für Carboxy steht. Ferner kann man eine Cyanogruppe R₅ zu Carbamoyl hydrolysieren.

Ferner kann man in Verbindung der Formel 1, worin R₁ und/oder R₂ Wasserstoff bedeutet, das Wasserstoffatom durch einen von Wasserstoff verschiedenen Rest R₁ und/oder R₂ ersetzen.

So kann man in üblicher Weise Niederalkanoyl einführen, beispielsweise durch Umsetzung mit einem reaktionsfähigen Niederalkansäurederivat, wie einem Niederalkansäurechlorid oder Niederalkansäurenitril, in Gegenwart von Aluminiumtrichlorid, bei Umsetzung mit einem Niederalkansäurenitril erforderlichenfalls in Gegenwart von Bortrichlorid, vorzugsweise in einem halogenierten Kohlenwasserstoff, erforderlichenfalls in der Siedehitze.

Weiterhin kann man in üblicher Weise einen Niederalkyl-, Niederalkenyl- oder Niederalkinylrest R₅ einführen, beispielsweise in Gegenwart eines basischen Kondensationsmittels, wie in Gegenwart von Tetrabutylammoniumjodid in Dimethylformamid.

Weiterhin kann man Niederalkenyl R₅ in üblicher Weise, insbesondere mittels Osmiumtetroxid in Dihydroxyniederalkyl überführen.

Auch kann man in erhaltene Verbindungen der Formel I, worin R₅ Wasserstoff ist, in üblicher Weise einen von Wasserstoff verschiedenen Rest einführen, beispielsweise niederalkylieren, niederalkanoylieren oder gegebenenfalls verestertes oder amidiertes Carboxy oder Carboxyniederalkyl einführen.

Erhaltene Salze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formel 1, einschließlich ihrer Salze, können auch in Form von Hydraten erhalten werden oder das zur Kristallisation verwendete Lösungsmittel einschließen.

Infolge der engen Beziehung zwischen den neuen Verbindungen in freier Form und in Form ihrer Salze sind vorstehend und nachfolgend unter den freien Verbindungen und ihren Salzen sinn- und zweckgemäß gegebenenfalls auch die entsprechenden Salze bzw. freien Verbindungen zu verstehen.

Erhaltene Diastereomerengemische und Racematgemische können auf Grund der physikalisch-chemischen Unterschiede der Bestandteile in bekannter Weise in die reinen Diastereomeren bzw. Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung des erhaltenen Diastereomerengemisches bzw. Racemates mit einer optisch aktiven Hilfsverbindung, z.B. entsprechend der in Verbindungen der Formel I enthaltenen sauren, basischen oder funktionell abwandelbaren Gruppen mit einer optisch aktiven Säure, Base oder einem optisch aktiven Alkohol, in Gemische diastereomerer Salze bzw. funktioneller Derivate, wie Ester, Trennung derselben in die Diastereomeren, aus denen das jeweils gewünschte Enantiomere in der jeweils üblichen Weise freigesetzt werden kann. Dafür geeignete Basen, Säuren bzw. Alkohole sind beispielsweise optisch aktive Alkaloidbasen, wie Strychnin, Cinchonin oder Brucin, oder D- oder L-(1-Phenyl)ethylamin, 3-Pipecolin, Ephedrin, Amphetamin und ähnliche synthetisch zugängliche Basen, optisch aktive Carbon- oder Sulfonsäuren, wie Chinasäure oder D- oder L-Weinsäure, D- oder L-Di-o-toluylweinsäure, D- oder L-Äpfelsäure, D- oder L-Mandelsäure, oder D- oder L-Camphersulfonsäure, bzw. optisch aktive Alkohole, wie Borneol oder D- oder L-(1-Phenyl)ethanol.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemäßen Verbindungen entwickelt wurden, insbesondere die zu den eingangs als bevorzugt gekennzeicheten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Die Erfindung betrifft gleichfalls pharmazeutische Präparate, die erfindungsgemäßen Verbindungen oder pharmazeutisch verwendbare Salze derselben als Wirkstoffe enthalten, sowie Verfahren zu ihrer Herstellung.

Bei den erfindungsgemäßen pharmazeutischen Präparaten, welche die erfindungsgemäßen Verbindung oder pharmazeutisch verwendbare Salze davon enthalten, handelt es sich um solche zur enteralen, wie oralen, ferner rektalen, und parenteralen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffes hängt von dem Alter und dem individuellen Zustand sowie von der Applikationsweise ab.

Die neuen pharmazeutischen Präparate enthalten z.B. von etwa 10 % bis etwa 80 %, vorzugsweise von etwa 20 % bis etwa 60 %, des Wirkstoffs. Erfindungsgemäße pharmazeutische Präparate zur enteralen bzw. parenteralen Verabreichung sind z.B. solche in Dosiseinheitsformen, wie Dragées, Tabletten, Kapseln oder Suppositorien, ferner Ampullen.

Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach.Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Traganth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fließ-, Fließregulierund Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaft-resistenten Überzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Ölen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetisch Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wässerlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die Dosierung des Wirkstoffes hängt von der Warmblüter-Spezies, dem Alter und dem individuellen Zustand sowie der Applikationsweise ab. Im Normalfall ist für einen etwa 75 kg schweren Patienten bei oraler Applikation eine ungefähre Tagesdosis von etwa 10 mg bis etwa 500 mg zu veranschlagen.

Die nachfolgen Beispiele dienen zur Illustration der Erfindung; Temperaturen sind in Celsiusgraden, Drucke in mbar angegeben.

### Beispiel 1:

2,0 g (4,07 mMol) 5-Amino-6,7-dichlor-chinoxalin-2,3-dion und 1,98 g (6,1 mMol) Fumarsäuremonoethylesterchlorid werden in 80 ml wasserfreiem Dioxan suspendiert und über Nacht unter Argon zum Rückfluß erhitzt. Man läßt auf Raumtemperatur abkühlen, filtriert das Reaktionsprodukt ab und wäscht nacheinander mit Dioxan und Diethylether. Nach dem Trocknen erhält man 2,56 g (6,88 mMol; 85 % d.Th.) 8,9-Dichlor-2,4,5-trioxo-2,3,5,6-tetrahydro-1 H,4H-1,3a,6-triaza-phenalen-3-ylessigsäureethylester vom Smp. > 300°.

### Beispiel 2:

492 mg (2 mMol) 5-Amino-6,7-dichlor-chinoxalin-2,3-dion und 2,07 g (10 mMol) Fumarsäuremonobenzylesterchlorid werden in 20 ml wasserfreiem Dioxan gelöst und über Nacht unter Argon zum Rückfluß erhitzt. Man läßt auf Raumtemperatur abkühlen, fügt 80 ml Diethylether hinzu, filtriert das Reaktionsprodukt ab und wäscht mit Diethylether. Das Rohprodukt wird in Essigsäureethylester aufgenommen und nochmals 10 Minuten zum Rückfluß erhitzt. Man filtriert heiß ab, wäscht mit Essigsäureethylester und Diethylether und läßt trocknen. Man erhält 490 mg (1,13 mMol; 56 % d.Th.) 8,9-Dichlor-2,4,5-trioxo-2,3,5,6-tetrahydro-1H,4H-1,3a,6-triaza-phenalen-3-ylessigsäurebenzylester in Form eines hellbraunen Festkörpers vom Smp. 295-298° (Zers.).

### Beispiel 3:

1,12 g ( 3 mMol) 8,9-Dichlor-2,4,5-trioxo-2,3,5,6-tetrahydro-1H,4H-1,3a,6-triazaphenalen-3-ylessigsäureethylester werden in 30 ml eines Gemisches von 2 Teilen Tetrahydrofuran und 1 Teil Wasser suspendiert und mit 630 mg (15 mMol) Lithiumhydroxidmonohydrat versetzt. Die erhaltene Lösung wird 60 Stunden bei Raumtemperatur gerührt. Das Tetrahydrofuran wird unter vermindertem Druck abgezogen. Es bildet sich eine geringfügige Mengen eines Niederschlages, der abfiltriert und verworfen wird. Man fügt unter Rühren 30 ml 1N Salzsäure hinzu, filtriert den ausgefällten weißen Niederschlag ab, wäscht nacheinander mit Wasser, Aceton und Diethylether und läßt trocknen. Man erhält 970 mg (2,82 mMol; 94% d.Th.) 8,9-Dichlor-2,4,5-trioxo-2,3,5,6-tetrahydro-1H,4H-1,3a,6-triazaphenalen-3-ylessigsäure in Form eines weißen Festkörpers vom Smp. > 300°.

### Beispiel 4:

688 mg (2 mMol) 8,9-Dichlor-2,4,5-trioxo-2,3,5,6-tetrahydro-1H,4H-1,3a,6-triazaphenalen-3-ylessigsäure, 279 mg (3 mMol) Anilin, 767 mg (4 mMol) N-(3-Dimethylamino)propyl-N'-ethyl-carbodiimid-hydrochlorid und 541 mg (4 mMol) N-Hydroxybenztriazol werden in 20 ml Dimethylformamid gelöst und bei Raumtemperatur über Nacht unter Argon gerührt. Dann wird das Reaktionsgemisch in 600 ml Wasser eingerührt.

Man läßt 10 Minuten nachrühren, filtriert ab, wäscht mit Wasser und läßt im Hochvakuum über Phosphorpentoxid trocknen. Man erhält 770 mg (1,84 mMol; 92% d.Th.) 8,9-Dichlor-2,4,5-trioxo-2,3,5,6-tetrahydro-1H,4H-1,3a,6-triaza-phenalen-3-yl-N-phenyl-acetamid in Form eines hellbraunen Feststoffes vom Smp. > 300°.

### Beispiel 5:

In analoger Weise wie in Beispiel 1 beschrieben erhält man 8,9-Dimethyl-2,4,5-trioxo-2,3,5,6-tetrahydro-1H,4H-13a,6-triaza-phenalen-3-ylessigsäureethylester vom Smp. > 300°.

### Beispiel 6:

Aus dem Ester gemäß Beispiel 5 erhält man in analoger Weise wie in Beispiel 3 beschrieben 8,9-Dimethyl-2,4,5-trioxo-2,3,5,6-tetrahydro-1H,4H-1,3a,6-triaza-phenalen-3-ylessigsäure vom Smp. > 300°.

### Beispiel 7:

Aus der Säure gemäß Beispiel 6 erhält man in analoger Weise wie in Beispiel 4 beschrieben 8,9-Dimethyl-2,4,5-trioxo-2,3,5,6-tetrahydro-1H,4H-1,3a,6-triaza-phenalen-3-ylessigsäure-N-phenyl-acetamid vom Smp. > 300°.

### Beispiel 8:

30 g (122 mMol) 5-Amino-6,7-dichlor-chinoxalin-2,3-dion werden mit 32.7 g (183 mMol) 4-Bromcrotonsäuremethylester in 100 ml Dimethylformamid unter Rühren 20 Stunden auf 100° erwärmt. Man läßt auf Raumtemperatur abkühlen, gießt in 2 Liter Eiswasser, läßt 15 Minuten rühren, filtriert ab, wäscht mit Wasser und trocknet. Man erhält 40.61 g (118 mMol; 97% d. Th.) 8,9-Dichlor-4,5-dioxo-2,3,5,6-tetrahydro-1H,4H-1,3a,6-triaza-phenalen-3-ylessigsäuremethylester vom Smp. 264-268°.

### Beispiel 9:

In analoger Weise wie in Beispiel 8 beschrieben erhält man die folgenden Verbindungen:
8,9-Dimethyl-4,5-dioxo-2,3,5,6-tetrahydro-1 H,4H-1,3a,6-triaza-phenalen-3-ylessigsäuremethylester, Smp. 303-306°;
8,9-Difluor-4,5-dioxo-2,3,5,6-tetrahydro-1 H,4H-1,3a,6-triaza-phenalen-3-ylessigsäuremethylester, Smp. > 250°;
4,5-Dioxo-2,3,5,6-tetrahydro-1H,4H-1,3a,6-triaza-phenalen-3-ylessigsäuremethylester, Smp. 244-247°;
8-Brom-4,5-dioxo-2,3,5,6-tetrahydro-1H,4H-1,3a,6-friaza-phenalen-3-ylessigsäuremethylester und
8-Trifluormethyl-4,5-dioxo-2,3,5,6-tetrahydro-1H,4H-1,3a,6-triaza-phenalen-3-ylessigsäuremethylester, Smp. 150-154°.

### Beispiel 10:

14.15 g (41.1 mMol) 8,9-Dichlor-4,5-dioxo-2,3,5,6-tetrahydro-1H,4H-1,3a,6-triaza-phenalen-3-ylessigsäuremethylester werden in 200 ml Methanol angeschlämmt, mit 100 ml wäßriger Natronlauge versetzt und 1 Stunde bei Raumtemperatur gerührt. Das Methanol wird unter vermindertem Druck abgezogen. Die wäßrige Phase wird zweimal mit je 60 ml Diethylether nachgewaschen und durch langsame Zugabe von 2n Salzsäure unter Rühren auf pH = 1 angesäuert. Der feste Niederschlag wird abgesaugt, mit Wasser gewaschen und getrocknet. Man erhält 11.5 (34.8 mMol, 85% d. Th.) 8,9-Dichlor-4,5-dioxo-2,3,5,6-tetrahydro-1 H,4H-1,3a,6-triaza-phenalen-3-ylessigsäure vom Smp.. 268-272°.

### Beispiel 11:

In analoger Weise wie in Beispiel 10 beschrieben erhält man die folgenden Verbindungen:
8,9-Dimethyl-4,5-dioxo-2,3,5,6-tetrahydro-1 H,4H-1,3a,6-triaza-phenalen-3-ylessigsäure, Smp. 285-287°;
8,9-Difluor-4,5-dioxo-2,3,5,6-tetrahydro-1H,4H-1,3a,6-triaza-phenalen-3-ylessigsäure, Smp. 239-245°;
4,5-Dioxo-2,3,5,6-tetrahydro-1H,4H-1,3a,6-triaza-phenalen-3-ylessigsäure, Smp. > 260°;
8-Brom-4, 5-dioxo-2, 3,5,6-tetrahydro-1H,4H-1,3a,6-triaza-phenalen-3-ylessigsäure, Smp. > 260° und
8-Trifluormethyl-4,5-dioxo-2,3,5,6-tetrahydro-1H,4H-1,3a,6-triaza-phenalen-3-ylessigsäure, Smp. ∼200°.

### Beispiel 12:

300 mg (0.91 mMol) 8,9-Dichlor-4,5-dioxo-2,3,5,6-tetrahydro-1H,4H-1,3a,6-triaza-phenalen-3-ylessigsäure, 0.12 ml (1.36 mMol) Anilin, 384 mg (1,82 mMol) N-(3-Dimethylamino)propyl-N'-ethyl-carbodiimid-hydrochlorid und 246 mg (1.82 mMol) N-Hydroxybenztriazol werden in 10 ml wasserfreiem Dimethylformamid 48 Stunden bei Raumtemperatur gerührt. Dann wird das Reaktionsgemisch in 250 ml Eiswasser eingerührt. Man filtiert ab, wäscht mit Wasser und läßt trocknen. Man erhält 310 mg (1,84 mMol; 84% d.Th.) 8,9-Dichlor-4,5-dioxo-2,3,5,6-tetrahydro-1H,4H-1,3a,6-triaza-phenalen-3-yl-N-phenyl-acetamid in Form eines hellbraunen Feststoffes vom Smp. > 300°.

### Beispiel 13:

In analoger Weise wie in Beispiel 12 beschrieben erhält man die folgenden Verbindungen:
8,9-Dimethyl-4,5-dioxo-2,3,5,6-tetrahydro-1 H,4H-1,3a,6-triaza-phenalen-3-N-phenylacetamid, Smp. > 300°;
8,9-Difluor-4,5-dioxo-2,3,5,6-tetrahydro-1 H,4H-1,3a,6-triaza-phenalen-3-N-phenyl-acetamid, Smp. > 260°;
4,5-Dioxo-2,3,5,6-tetrahydro-1H,4H-1,3a,6-triaza-phenalen-3-N-phenyl-acetamid, Smp. 284-290°;
8-Brom-4,5-dioxo-2,3,5,6-tetrahydro-1H,4H-1,3a,6-triaza-phenalen-3-phenyl-acetamid, Smp. > 250° und
8-Trifluormethyl-4,5-dioxo-2,3,5,6-tetrahydro-1H,4H-1,3a,6-triaza-phenalen-3-phenylacetamid, Smp. > 250°.

### Beispiel 14:

In analoger Weise wie in Beispiel 12 beschrieben erhält man ausgehend von 8,9-Dichlor-4,5-dioxo-2,3,5,6-tetrahydro-1H,4H-1,3a,6-triaza-phenalen-3-ylessigsäure auch ihre nachstehend aufgeführten Amide:
8,9-Dichlor-4,5-dioxo-2,3,5,6-tetrahydro-1H,4H-1,3a,6-triaza-phenalen-3-yl-N-benzylacetamid, Smp. 255-260°;
8,9-Dichlor-4,5-dioxo-2,3,5,6-tetrahydro-1H,4H-1,3a,6-triaza-phenalen-3-yl-N-(2-phenylethyl)-acetamid, Smp. 170-175°;
8,9-Dichlor-4,5-dioxo-2,3,5,6-tetrahydro-1H,4H-1,3a,6-triaza-phenalen-3-yl-N-(2-hydroxyethyl)-acetamid, Smp. >250°;
8,9-Dichlor-4,5-dioxo-2,3,5,6-tetrahydro-1H,4H-1,3a,6-triaza-phenalen-3-yl-N-(ethoxycarbonylmethyl)-acetamid, Smp. > 250°;
8,9-Dichlor-4,5-dioxo-2,3,5,6-tetrahydro-1H,4H-1,3a,6-triaza-phenalen-3-yl-N-(carboxymethyl)-acetamid, Smp. > 250°;
8,9-Dichlor-4,5-dioxo-2,3,5,6-tetrahydro-1H,4H-1,3a,6-triaza-phenalen-3-yl-N-(3-trifluormethylphenyl)-acetamid, Smp. > 300°;
8,9-Dichlor-4,5-dioxo-2,3,5,6-tetrahydro-1H,4H-1,3a,6-triaza-phenalen-3-yl-N-(4-chlorphenyl)-acetamid, Smp. > 300°;
8,9-Dichlor-4,5-dioxo-2,3,5,6-tetrahydro-1H,4H-1,3a,6-triaza-phenalen-3-yl-N-(4-methylphenyl)-acetamid, Smp. > 300°;
8,9-Dichlor-4,5-dioxo-2,3,5,6-tetrahydro-1H,4H-1,3a,6-triaza-phenalen-3-yl-N-(4-methoxyphenyl)-acetamid, Smp. > 300°;
8,9-Dichlor-4,5-dioxo-2,3,5,6-tetrahydro-1H,4H-1,3a,6-triaza-phenalen-3-yl-N-(4-phenoxyphenyl)-acetamid, Smp. > 300°;
8,9-Dichlor-4,5-dioxo-2,3,5,6-tetrahydro-1H,4H-1,3a,6-triaza-phenalen-3-yl-N-(3-nitrophenyl)-acetamid, Smp. > 300°;
8,9-Dichlor-4,5-dioxo-2,3,5,6-tetrahydro-1H,4H-1,3a,6-triaza-phenalen-3-yl-N-(4-ethoxycarbonylphenyl)acetamid, Smp. > 300°;
8,9-Dichlor-4,5-dioxo-2,3,5,6-tetrahydro-1H,4H-1,3a,6-triaza-phenalen-3-yl-N-(4-trifluormethylphenyl)-acetamid, Smp. > 300°;
8,9-Dichlor-4,5-dioxo-2,3,5,6-tetrahydro-1H,4H-1,3a,6-triaza-phenalen-3-yl-N-benzyloxyacetamid, Smp. 186-189°;
8,9-Dichlor-4,5-dioxo-2,3,5,6-tetrahydro-1H,4H-1 ,3a,6-triaza-phenalen-3-yl-N-tertiärbutyloxy-acetamid, Smp. > 300°;
8,9-Dichlor-4,5-dioxo-2,3,5,6-tetrahydro-1H,4H-1,3a,6-triaza-phenalen-3-yl-N-(3-biphenyl)acetamid, Smp. > 300°;
8,9-Dichlor-4,5-dioxo-2,3,5,6-tetrahydro-1H,4H-1,3a,6-triaza-phenalen-3-yl-N-(3-phenoxyphenyl)-acetamid, Smp.. 262-266°;
8,9-Dichlor-4,5-dioxo-2,3,5,6-tetrahydro-1H,4H-1,3a,6-triaza-phenalen-3-yl-N, N-dimethylacetamid, Smp. > 300°;
8,9-Dichlor-4,5-dioxo-2,3,5,6-tetrahydro-1H,4H-1,3a,6-triaza-phenalen-3-yl-N-butyl-acetamid;
8,9-Dichlor-4,5-dioxo-2,3,5,6-tetrahydro-1H,4H-1 ,3a,6-triaza-phenalen-3-yl-N-tertiärbutylacetamid, Smp. > 260°;
8,9-Dichlor-4,5-dioxo-2,3,5,6-tetrahydro-1H,4H-1,3a,6-triaza-phenalen-3-yl-N,N-dibutylacetamid;
8,9-Dichlor-4,5-dioxo-2,3,5,6-tetrahydro-1H,4H-1,3a,6-triaza-phenalen-3-yl-N-(adamantan-1-yl)-acetamid, Smp. > 260°;
8,9-Dichlor-4,5-dioxo-2,3,5,6-tetrahydro-1H,4H-1,3a,6-triaza-phenalen-3-yl-N-methyl-acetamid, Smp. > 280°;
7,8-Dichlor-4-{2-oxo-2-[4-(2-oxo-imidazolidin-1-yl)piperidin-1-yl]ethyl}-5,6-dihydro-1H,4H,1,3a,6-triaza-phenalen-2,3-dion, Smp. > 260°;
8,9-Dichlor-4,5-dioxo-2,3,5,6-tetrahydro-1H,4H-1,3a,6-triaza-phenalen-3-yl-N-methyl-N-phenyl-acetamid, Smp. > 280°,
8,9-Dichlor-4,5-dioxo-2,3,5,6-tetrahydro-1H,4H-1,3a,6-triaza-phenalen-3-yl-N-[2-(2-oxoimidazolidin-1-yl]ethyl]-acetamid, Smp. > 260° und
8,9-Dichlor-4,5-dioxo-2,3,5,6-tetrahydro-1H,4H-1,3a,6-triaza-phenalen-3-yl-N-(2-morpholinoethyl)-acetamid, Smp. > 280°.

### Beispiel 15:

Zu einer Lösung von 2.57 g (9.33 mMol) 4,5-Dioxo-2,3,5,6-tetrahydro-1H,4H-1,3a,6-triaza-phenalen-3-ylessigsäuremethylester in 40 ml 1,2-Dichlorethan werden bei 0° unter Argon 20.5 ml einer 1-molaren Lösung von Bortrichlorid in Heptan zugegeben. Nach 10 Minuten fügt man zunächst 2.74 g (20.5 mMol) Aluminiumtrichlorid und dann 2.75 g (50 mMol) Propionitril hinzu. Man läßt auf Raumtemperatur erwärmen und erhitzt dann 3 Stunden zum Rückfluß. Mann läßt auf Raumtemperatur abkühlen, fügt 50 ml 2N Salzsäure hinzu und erhitzt nochmals 1 Stunde zum Rückfluß. Nach erneutem Abkühlen auf Raumtemperatur gibt man 100 ml Wasser hinzu, schüttelt zweimal mit je 100 ml Trichlormethan aus, wäscht mit gesättigter Kochsalzlösung und trocknet über Natriumsulfat. Man dampft zur Trockne ein, nimmt in 15 ml Tetrahydrofuran auf, gibt 30 ml N Natronlauge hinzu und läßt über Nacht rühren. Dann gibt man 20 ml Wasser hinzu, wäscht zweimal mit je 50 ml Diethylether, säuert mit verdünnter Salzsäure auf pH = 2 an und schüttelt zweimal mit je 100 ml Essigsäureethylester aus. Die vereinigten Auszüge werden über Natriumsulfat getrocknet und eingedampft. Der Eindampfrückstand wird in 5 ml wasserfreiem Dimethylformamid aufgenommen, mit 211 mg (2.27 mMol) Anilin, 435 mg (2.27 mMol) N-(3-Dimethylamino)propyl-N'-ethyl-carbodiimid-hydrochlorid und 348 mg (2.27 mMol) N-Hydroxybenztriazol versetzt und 48 Stunden bei Raumtemperatur gerührt. Man gießt in 400 ml Wasser, saugt ab, wäscht mit Wasser und trocknet. Man erhält 9-Propionyl-4,5-dioxo-2,3,5,6-tetrahydro-1H,4H-1,3a,6-triaza-phenalen-3-yl-N-phenyl-acetamid, Smp. > 300°.

### Beispiel 16:

Zu einer Mischung von 0.88 g (11 mMol) Ammoniumnitrat, und 2.75 g (10 mMol) 4,5-Dioxo-2,3,5,6-tetrahydro-1H,4H-1,3a,6-triaza-phenalen-3-ylessigsäuremethyl-ester und 60 ml Trichlormethan werden in der Siedehitze unter Stickstoff 16.8 g (80 mMol) Trifluoressigsäureanhydrid zugetropft.Man erhitzt weitere 2 Stunden zum Rückfluß, läßt auf Raumtemperatur abkühlen, gießt in Eiswasser, schüttelt mit Essigsäureethylester aus, wäscht mit Wasser und gesättigter Kochsalzlösung, trocknet über Magnesiumsulfat und dampft ein. Der Eindampfrückstand wird an Kieselgel mit Essigsäureethylester als Laufmittel chromatographiert. Man erhält 1.07 g 9-Nitro-4,5-dioxo-2,3,5,6-tetrahydro-1 H,4H-1,3a,6-triaza-phenalen-3-ylessigsäuremethylester und 0.43 g 7-Nitro-4,5-dioxo-2,3,5,6-tetrahydro-1H,4H-1,3a,6-triaza-phenalen-3-ylessigsäuremethylester.

### Beispiel 17:

In analoger Weise wie in Beispiel 10 beschrieben erhält man aus 9-Nitro-4,5-dioxo-2,3,5,6-tetrahydro-1 H,4H-1,3a,6-triaza-phenalen-3-ylessigsäuremethylester und 7-Nitro-4,5-dioxo-2,3,5,6-tetrahydro-1H,4H-1,3a,6-triaza-phenalen-3-ylessigsäuremethylester 9-Nitro-4,5-dioxo-2,3,5,6-tetrahydro-1H,4H-1,3a,6-triaza-phenalen-3-ylessigsäure, Smp. > 250° und 7-Nitro-4,5-dioxo-2,3,5,6-tetrahydro-1H,4H-1,3a,6-triaza-phenalen-3-ylessigsäure, Smp. >250°.

### Beispiel 18:

In analoger Weise wie in Beispiel 12 beschrieben erhält man aus 9-Nitro-4,5-dioxo-2,3,5,6-tetrahydro-1H,4H-1,3a,6-triaza-phenalen-3-ylessigsäure, Smp. > 250° und 7-Nitro-4,5-dioxo-2,3,5,6-tetrahydro-1H,4H-1,3a,6-triaza-phenalen-3-ylessigsäure 9-Nitro-4,5-dioxo-2,3,5,6-tetrahydro-1H,4H-1,3a,6-triaza-phenalen-3-yl-N-phenyl-acetamid, Smp. > 250° und 7-Nitro-4, 5-dioxo-2,3,5,6-tetrahydro-1H,4H-1 ,3a,6-triaza-phenalen-3-yl-N-phenylacetamid, Smp.. 220-240° (Zers.).

### Beispiel 19:

2.31 g (6.52 mMol) 8-Brom-4,5-dioxo-2,3,5,6-tetrahydro-1H,4H-1,3a,6-triazaphenalen-3-ylessigsäuremethylester 13.04 mMol Allylbromid und 6,52 mMol Tetrabutylammoniumjodid werden in 20 ml wasserfreiem Dimethylformamid 5 Tage auf 100° erhitzt. Das Dimethylformamid wird unter vermindertem Druck abgezogen und der Rückstand an Kieselgel mit Essigsäureethylester als Laufmittel chromatographiert. Man erhält 1.81 g 1-Allyl-8-brom-4,5-dioxo-2,3,5,6-tetrahydro-1H,4H-1,3a,6-triaza-phenalen-3-ylessig-säuremethylester.

### Beispiel 20:

In analoger Weise wie in Beispiel 10 beschrieben erhält man aus 1-Allyl-8-brom-4,5-dioxo-2,3,5,6-tetrahydro-1H,4H-1,3a,6-triaza-phenalen-3-ylessigsäuremethylester 1-Allyl-8-brom-4,5-dioxo-2,3,5,6-tetrahydro-1 H,4H-1,3a,6-triaza-phenalen-3-ylessigsäure, Smp. 243-245°.

### Beispiel 21:

In analoger Weise wie in Beispiel 12 beschrieben erhält man aus 1-Allyl-8-brom-4,5-dioxo-2,3,5,6-tetrahydro-1H,4H-1,3a,6-triaza-phenalen-3-ylessigsäure 1-Allyl-8-brom-4,5-dioxo-2,3,5,6-tetrahydro-1H,4H-1,3a,6-triaza-phenalen-3-yl-N-phenyl-acetamid, Smp. > 250°.

### Beispiel 22:

219 mg (0.48 mMol) 1-Allyl-8-brom-4,5-dioxo-2,3,5,6-tetrahydro-1H,4H-1,3a,6-triaza-phenalen-3-yl-N-phenyl-acetamid, 75 mg (0.55 mMol) N-Methylmorpholin-N-oxid und 0.4 ml einer 4 %-igen wäßrigen Lösung von Osmiumtetroxid werden in einem Gemisch aus 7 ml Wasser, 7 ml Dioxan und 1 ml Tertiärbutanol 16 Stunden gerührt. Die organischen Lösungsmittel werden abgezogen. Der Rückstand wird mit kaltem Wasser versetzt, abgesaugt, mit Wasser gewaschen und getrocknet. Man erhält 85 mg 8-Brom-1-(2,3-dihydroxypropyl)-4,5-dioxo-2,3,5,6-tetrahydro-1H,4H-1,3a,6-triaza-phenalen-3-yl-N-phenyl-acetamid, Smp. > 250°.

### Beispiel 23:

2.49 g (10.1 mMol) 5-Amino-6,7-dichlor-chinoxalin-2,3-dion und 2.70 g (12.62 mMol) E-1.4-Dibrom-2-buten werden in 60 ml wasserfreiem Dimethylformamid 48 Stunden unter Argon auf 100° erhitzt. Man läßt auf Raumtemperatur abkühlen, gießt in 700 ml Eiswasser, läßt 15 Minuten rühren, filtriert ab, wäscht mit Wasser, trocknet und nimmt in 50 ml Aceton auf. Man läßt 20 Minuten am Rückfluß sieden, auf Raumtemperatur abkühlen, filtriert ab, wäscht mit Aceton und trocknet. Man erhält 1.50 g (5.03 mMol, 50% d. Th.) 7,8-Dichlor-4-vinyl-5,6-dihydro-1H,4H-1,3a,6-triaza-phenalen-2,3-dion, Smp. > 260°.

### Beispiel 24:

570 mg (2.32 mMol) 5-Amino-6,7-dichlor-chinoxalin-2,3-dion und 523 mg (4.63 mMol) Chloracetylchlorid werden in 25 ml Dioxan 16 Stunden unter Rühren zum Rückfluß erhitzt. Man läßt auf Raumtemperatur abkühlen, filtriert ab, wäscht mit Diethylether und trocknet. Der erhaltene Festkörper wird mit 490 mg (3.54 mMol) Kaliumcarbonat in 40 ml wasserfreiem Dimethylformamid angeschlämmt und 16 Stunden bei Raumtemperatur gerührt. Man gießt in ein Gemisch aus 600 ml Wasser und 15 ml konzentrierter Salzsäure, filtriert ab, wäscht mit Wasser und Aceton und schlämmt in 50 ml Ethanol an. Man erhitzt 30 Minuten zum Rückfluß, filtriert heiß ab, wäscht mit Ethanol und Diethylether und trocknet.

Man erhält 310 mg (1.08 mMol, 47% d. Th.) 7,8-Dichlor-1H,6H-1,3a,6-triaza-phenalen-2,3,5-trion.

### Beispiel 25:

2.45 g (10 mMol) 5-Amino-6,7-dichlor-chinoxalin-2,3-dion und 7.7 g 3-Bromprop-1-enphosphonsäurediethylester werden in 40 ml wasserfreiem Dimethylformamid gelöst und 4 Tage auf 90° erhitzt. Man läßt auf Raumtemperatur abkühlen, gießt in 500 ml Eiswasser, fügt 100 ml N-Natronlauge hinzu und wäscht zweimal mit je 500 ml Dichlormethan. Die wäßrige Phase wird mit verdünnter Salzsäure auf pH 1 angesäuert, das ausgefällte Niederschlag wird abfiltriert, mit Wasser gewaschen und getrocknet. Der erhaltene Festkörper wird bei 0° in 20 ml Dichlormetahn suspendiert und mit 580 mg (5.7 mMol) Trimethylbromsilan versetzt. Man läßt über Nacht bei Raumtemperatur rühren und dampft unter vermindertem Druck ein. Der Eindampfrückstand wird mit 10 ml Methanol angeschlämmt und in 100 ml 0.1N Salzsäure gegossen. Man filtiriert ab und erhält nach Waschen mit Wasser und Trocknen 250 mg 8,9-Dichlor-4,5-dioxo-2,3,5,6-tetrahydro-1H,4H-1,3a,6-triaza-phenalen-3-ylmethanphosphonsäure vom Smp. > 250

### Beispiel 26:

Tabletten, enthaltend je 50 mg 8,9-Dichlor-2,4,5-trioxo-2,3, 5,6-tetrahydro- 1H,4H-1,3a,6-triaza-phenalen-3-ylessigsäure oder ein Salz, z.B. das Natriumsalz, davon können wie folgt hergestellt werden:

| Zusammensetzung (10000 Tabletten) | |
|---|---|
| Wirkstoff 500.0 g | |
| Laktose | 500.0 g |
| Kartoffelstärke | 352.0 g |
| Gelatine | 8.0 g |
| Talk | 60.0 g |
| Magnesiumpstearat | 10.0 g |
| Siliciumdioxid (hochdispers) | 20.0 g |
| Ethanol | q.s. |

Der Wirkstoff wird mit der Laktose und 292 g Kartoffelstärke vermischt, die Mischung mit einer ethanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und preßt das Gemisch zu Tabletten von je 145,0 mg Gewicht und 50,0 mg Wirkstoffgehalt, die gewünschtenfalls mit Teilkerben zur feineren Anpassung der Dosierung versehen sein können.

### Beispiel 27:

Eine sterilfiltrierte wäßrige Gelatine-Lösung mit 20 % Cyclodextrinen als Lösungsvermittler, enthaltend je 3 mg 8,9-Dichlor-2,4,5-trioxo-2,3,5,6-tetrahydro-1H,4H-1,3a,6-triaza-phenalen-3-ylessigsäure oder ein Salz, z.B. das Natriumsalz, davon als Wirkstoff wird unter Erwärmen mit einer sterilen Gelatinelösung, welche als Konservierungsmittel Phenol enthält, unter aseptischen Bedingungen so vermischt, dass 1.0 ml Lösung die folgende Zusammensetzung hat:

| | |
|---|---|
| Wirkstoff | 3 mg |
| Gelatine | 150.0 mg |
| Phenol | 4.7 mg |
| dest. Wasser mit 20 % Cyclodextrinen als Lösungsvermittler | 1.0 ml |

### Beispiel 28:

Zur Herstellung einer sterilen Trockensubstanz zur Injektion, enthaltend je 5 mg 8,9-Dichlor-2,4,5-trioxo-2,3,5,6-tetrahydro-1H,4H-1,3a,6-triaza-phenalen-3-ylessigsäure oder ein Salz, z.B. das Natriumsalz, davon löst man 5 mg einer der in den vorausgehenden Beispielen genannten Verbindungen der Formel I als Wirkstoff in 1 ml einer wäßrigen Lösung mit 20 mg Mannit und 20 % Cyclodextrinen als Lösungsvermittler. Die Lösung wird sterilfiltriert und unter aseptischen Bedingungen in eine 2 ml-Ampulle gefüllt, tiefgekühlt und lyophilisiert. Vor dem Gebrauch wird das Lyophilisat in 1 ml destilliertem Wasser oder 1 ml physiologischer Kochsalzlösung gelöst. Die Lösung wird intramuskulär oder intravenös angewendet. Diese Formulierung kann auch in Doppelkammerspritzampullen abgefüllt werden.

### Beispiel 29:

Für die Herstellung von 10 000 Lacktabletten, enthaltend je 100 mg 8,9-Dichlor-2,4,5-trioxo-2,3,5,6-tetrahydro-1H,4H-1,3a,6-triaza-phenalen-3-ylessigsäure oder ein Salz, z.B. das Natriumsalz, davon können wie folgt hergestellt werden:

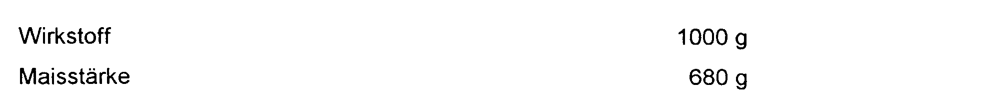

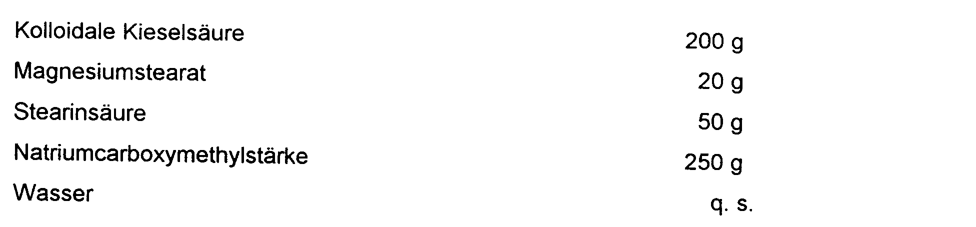

Ein Gemisch von einer der in den vorausgehenden Beispielen genannten Verbindungen der Formel I als Wirkstoff, 50 g Maisstärke und der kolloidalen Kieselsäure wird mit Stärkekleister aus 250 g Maisstärke und 2.2 kg entmineralisiertem Wasser zu einer feuchten Masse verarbeitet. Diese wird durch ein Sieb von 3 mm Maschenweite getrieben und bei 45° während 30 Minuten. im Wirbelschichttrockner getrocknet. Das getrocknete Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

### Beispiel 30:

In analoger Weise wie in den Beispielen 26 bis 29 beschrieben kann man ferner pharmazeutische Präparate enthaltend eine andere Verbindung gemäß einem der Beispielen 1 bis 25 herstellen.

## Patentansprüche

1. Neue azaaliphatisch überbrückte Chinoxalin-2,3-dione der Formel I 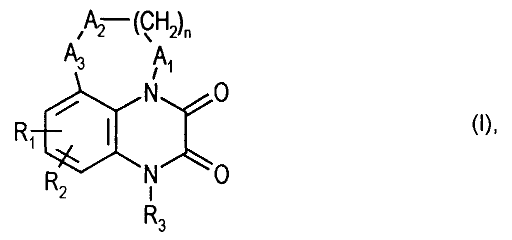 worin
A₁ Niederalkyliden oder eine Gruppe der Formel >CH-A₄-R₄ (Ia) darstellt,
A₂ Niederalkyliden oder eine Gruppe der Formeln >CH-A₄-R₄ (Ia) oder >C=O (Ib) oder >CH(OH)-A₅-R₄ (Ic) bedeutet,
A₃ eine Gruppe der Formel >N-A₆-R₅ (Id) darstellt,
A₄ für Niederalkylen steht,
A₅ und A₆ unabhängig voneinander Niederalkylen oder eine direkte Bindung darstellen,
n für 0 oder 1 steht,
R₁ und R₂ unabhängig voneinander Wasserstoff, gegebenenfalls durch Niederalkyl und/oder Niederalkanoyl substituiertes Amino, Nitro, Niederalkanoyl, gegebenenfalls verethertes Hydroxy, gegebenenfalls verestertes Carboxy, Carbamoyl, Cyano, gegebenenfalls halogeniertes Niederalkyl oder Halogen bedeuten,
R₃ Wasserstoff oder Hydroxy bedeutet,
R₄ gegebenenfalls verestertes oder amidiertes Carboxy, gegebenenfalls verestertes Phosphono oder 5-Tetrazolyl darstellt und
R₅ Wasserstoff, Niederalkyl, Niederalkenyl, Niederalkinyl, Aryl, Niederalkanoyl, gegebenenfalls verestertes oder amidiertes Carboxy, Cyano, 5-Tetrazolyl, gegebenenfalls verethertes oder verestertes Hydroxy oder Dihydroxyniederalkyl bedeutet,
und ihre Salze.

2. Eine Verbindung gemäß Anspruch 1 der Formel I, worin
A₁ Methylen oder eine Gruppe der Formel >CH-A₄-R₄ (Ia) darstellt,
A₂ Niederalkyliden oder eine Gruppe der Formel >C=O (Ib) ist,
A₃ eine Gruppe der Formel >N-A₆-R₅ (Id) bedeutet,
A₄ für Niederalkylen steht,
A₆ Niederalkylen oder eine direkte Bindung darstellt,
n für 0 oder 1 steht,
R₁ und R₂ unabhängig voneinander Wasserstoff, gegebenenfalls durch Niederalkyl und/oder Niederalkanoyl substituiertes Amino, gegebenenfalls verethertes Hydroxy, gegebenenfalls halogeniertes Niederalkyl oder Halogen bedeuten,
R₃ Wasserstoff oder Hydroxy bedeutet,
R₄ gegebenenfalls verestertes oder amidiertes Carboxy darstellt und
R₅ Wasserstoff, Aryl, Niederalkanoyl, gegebenenfalls verestertes oder amidiertes Carboxy, Cyano oder gegebenenfalls verethertes Hydroxy bedeutet,
oder ein Salz davon.

3. Eine Verbindung gemäß Anspruch 1 der Formel I, worin
A₁ Niederalkyliden oder eine Gruppe der Formel >CH-A₄-R₄ (Ia) darstellt,
A₂ Niederalkyliden oder eine Gruppe der Formeln >CH-A₄-R₄ (la) oder >C=O (Ib) oder >CH(OH)-A₅-R₅ (Ic) bedeutet,
A₃ eine Gruppe der Formel >N-A₆-R₅ (Id) darstellt,
A₄ für Niederalkylen steht,
A₅ und A₆ unabhängig voneinander Niederalkylen oder eine direkte Bindung darstellen,
n für 0 oder 1 steht,
R₁ und R₂ unabhängig voneinander Wasserstoff, Nitro, Niederalkanoyl, Amino, Niederalkylamino, Niederalkanoylamino, Diniederalkylamino oder N-Niederalkyl-N-niederalkanoylamino, Carboxy, Niederalkoxycarbonyl, Carbamoyl, Cyano, Hydroxy, Niederalkoxy, Niederalkenyloxy, Niederalkinyloxy, Niederalkyl, Polyhalogenniederalkyl oder Halogen bedeuten,
R₃ Wasserstoff oder Hydroxy bedeutet,
R₄ Carboxy, Niederalkoxycarbonyl, Carboxyniederalkoxycarbonyl, Niederalkoxycarbonylniederalkoxycarbonyl, Niederalkanoyloxyniederalkoxycarbonyl, unsubstituiertes oder durch Niederalkyl, Niederalkoxy, Hydroxy, Halogen und/oder Trifluormethyl substituiertes Phenylniederalkoxycarbonyl, Carbamoyl, Niederalkylcarbamoyl, Hydroxyniederalkylcarbamoyl, Diniederalkylcarbamoyl, Diniederalkylaminoniederalkylcarbamoyl, Aminoniederalkylaminoniederalkylcarbamoyl, 2-Oxoimidazolidin-1-ylniederalkylcarbamoyl, Aminoniederalkylaminoniederalkylencarbamoyl, 2-Oxoimidazolidin-1-ylniederalkylencarbamoyl, Oxaniederalkylenaminoniederalkylcarbamoyl, unsubstituiertes oder durch Carboxy oder Niederalkoxycarbonyl substituiertes Cycloalkylcarbamoyl, Cycloalkylniederalkylcarbamoyl, unsubstituiertes oder durch Niederalkyl, Niederalkoxy, Hydroxy, Halogen und/oder Trifluormethyl substituiertes Phenylniederalkylcarbamyol, unsubstituiertes oder durch Niederalkyl, Niederalkoxy, Hydroxy, Halogen, Nitro, Carboxy, Niederalkoxycarbonyl, Phenyl, Phenyloxy und/oder Trifluormethyl substituiertes N-Phenyl- oder N-Niederalkyl-N-phenylcarbamoyl, N-Hydroxycarbamoyl, N-Niederalkoxycarbamoyl N-Niederalkenyloxycarbamoyl, unsubstituiertes oder durch Niederalkyl, Niederalkoxy, Hydroxy, Halogen und/oder Trifluormethyl substituiertes N-Phenylniederalkoxy- oder N-Phenylniederalkenyloxycarbamoyl, Phophono, Niederalkylphosphono, Diniederalkylphosphono oder Triniederalkylphosphono oder 5-Tetrazolyl darstellt und
R₅ Wasserstoff, Niederalkyl, Niederalkenyl, Niederalkinyl, unsubstituiertes oder durch Niederalkyl, Niederalkoxy, Hydroxy, Halogen und/oder Trifluormethyl substituiertes Phenyl, Niederalkanoyl, Carboxy, Niederalkoxycarbonyl unsubstituiertes oder durch Niederalkyl, Niederalkoxy, Hydroxy, Halogen und/oder Trifluormethyl substituiertes Phenylniederalkoxycarbonyl, Carbamoyl, Niederalkylcarbamoyl, Diniederalkylcarbamoyl, unsubstituiertes oder durch Niederalkyl, Niederalkoxy, Hydroxy, Halogen, Nitro, Carboxy, Niederalkoxycarbonyl, Phenyl, Phenyloxy und/oder Trifluormethyl substituiertes N-Phenyl- oder N-Niederalkyl-N-phenylcarbamoyl, Cyano, Tetrazolyl, Hydroxy, Niederalkanoyloxy, Niederalkoxy, Niederalkenyloxy, Niederalkinyloxy oder Dihydroxyniederalkyl bedeutet, oder ein Salz davon.

4. Eine Verbindung gemäß Anspruch 1 der Formel I, worin
A₁ C₁-C₄-Alkyliden, wie Methylen, oder eine Gruppe der Formel >CH-A₄-R₄ (Ia) darstellt,
A₂ C₁-C₄-Alkyliden, wie Methylen, oder eine Gruppe der Formeln >CH-A₄-R₄ (Ia) oder >C=O (Ib) bedeutet,
A₃ eine Gruppe der Formel >N-A₆-R₅ (Id) darstellt,
A₄ für C₁-C₄-Alkylen steht,
A₅ und A₆ unabhängig voneinander C₁-C₄-Alkylen oder eine direkte Bindung darstellen,
n für 0 steht,
R₁ und R₂ unabhängig voneinander Wasserstoff, Nitro, C₁-C₇-Alkanoyl, Amino, C₁-C₇-Alkylamino, C₁-C₇-Alkanoylamino, Di-C₁-C₄-alkylamino, N-C₁-C₄-Alkyl-N-C₁-C₇-alkanoylamino, Hydroxy, Carboxy, C₁-C₄-Alkoxycarbonyl, Carbamoyl, Cyano, C₁-C₄-Alkoxy, C₂-C₄-Alkenyloxy, C₂-C₄-Alkinyloxy, C₁-C₄-Alkyl, Trifluormethyl oder Halogen bedeuten,
R₃ Wasserstoff oder Hydroxy bedeutet,
R₄ Carboxy, C₁-C₄-Alkoxycarbonyl, Carboxy-C₁-C₄-alkoxycarbonyl, C₁-C₄-Alkoxycarbonyl-C₁-C₄-alkoxycarbonyl, C₁-C₄-Alkanoyloxy-C₁-C₄-alkoxycarbonyl, unsubstituiertes oder durch C₁-C₄-Alkyl, C₁-C₄-Alkoxy, Hydroxy, Halogen und/oder Trifluormethyl substituiertes Phenyl-C₁-C₄-alkoxycarbonyl, Carbamoyl, C₁-C₇-Alkylcarbamoyl, Hydroxy-C₁-C₄-alkylcarbamoyl, Di-C₁-C₄-alkylcarbamoyl, Di-C₁-C₄-alkylamino-C₁-C₄-alkylcarbamoyl, Amino-C₁-C₄-alkylamino-C₁-C₄-alkylcarbamoyl, 2-Oxoimidazolidin-1-yl-C₁-C₄-alkylcarbamoyl, Amino-C₁-C₄-alkylamino-C₁-C₄-alkylencarbamoyl, 2-Oxoimidazolidin-1-ylniederalkylencarbamoyl, Oxa-C₁-C₄-alkylenamino-C₁-C₄-alkylcarbamoyl, unsubstituiertes oder durch Carboxy oder C₁-C₄-Alkoxycarbonyl substituiertes Cycloalkylcarbamoyl, Adamantylcarbamoyl, Cycloalkyl-C₁-C₄-alkylcarbamoyl, unsubstituiertes oder durch C₁-C₄-Alkyl, C₁-C₄-Alkoxy, Hydroxy, Halogen und/oder Trifluormethyl substituiertes Phenyl-C₁-C₄-alkylcarbamoyl, unsubstituiertes oder durch C₁-C₄-Alkyl, C₁-C₄-Alkoxy, Hydroxy, Halogen, Nitro, Carboxy, C₁-C₄-Alkoxycarbonyl, Phenyl, Phenyloxy und/oder Trifluormethyl substituiertes N-Phenyl- oder N-C₁-C₄-Alkyl-N-phenyl-carbamoyl, N-Hydroxycarbamoyl, N-C₁-C₄-Alkoxycarbamoyl, unsubstituiertes oder durch C₁-C₄-Alkyl, C₁-C₄-Alkoxy, Hydroxy, Halogen und/oder Trifluormethyl substituiertes N-Phenyl-C₁-C₄-alkoxycarbamoyl, Phosphono, C₁-C₇-Alkylphosphono, Di-C₁-C₇-Alkylphosphono, Tri-C₁-C₇-Alkylphosphono oder 5-Tetrazolyl darstellt und
R₅ Wasserstoff, C₁-C₄-Alkyl, wie Methyl, C₂-C₄-Alkenyl, wie Allyl, C₂-C₄-Alkinyl, wie Propargyl, unsubstituiertes oder durch C₁-C₄-Alkyl, wie Methyl, C₁-C₄-Alkoxy, wie Methoxy, Hydroxy, Halogen, wie Chlor, und/oder Trifluormethyl substituiertes Phenyl, C₁-C₄-Alkanoyl, wie Acetyl, Carboxy, C₁-C₄-Alkoxycarbonyl, wie Methoxy- oder Ethoxycarbonyl, Carbamoyl, C₁-C₄-Alkylcarbamoyl, wie Methylcarbamoyl, Di-C₁-C₄-alkylcarbamoyl, wie Dimethylcarbamoyl, unsubstituiertes oder durch C₁-C₄-Alkyl, wie Methyl, C₁-C₄-Alkoxy, Wie Methoxy, Hydroxy, Halogen, wie Fluor oder Chlor, Nitro, Carboxy, C₁-C₄-Alkoxycarbonyl, wie Methoxycarbonyl, Phenyl, Phenyloxy und/oder Trifluormethyl substituiertes N-Phenyl- oder N-C₁-C₄-Alkyl-N-phenyl-carbamoyl, wie N-Methyl-N-phenyl-carbamoyl, Cyano, Tetrazolyl oder Dihydroxy-C₂-C₄-alkyl, wie 2,3-Dihydroxypropyl, bedeutet, oder ein Salz davon.

5. Eine Verbindung gemäß Anspruch 1 der Formel 1, worin
A₁ eine Gruppe der Formel >CH-A₄-R₄ (Ia) darstellt,
A₂ C₁-C₄-Alkyliden, wie Methylen, 1,1-Ethyliden, 1,1-oder 2,2- Propyliden oder 1,1-oder 2,2-Butyliden, oder Carbonyl ist,
A₃ eine Gruppe der Formel >N-A₅-R₅ (Ic) bedeutet,
A₄ für geradkettiges oder verzweigtes C₁-C₄-Alkylen, wie Methylen, 1,1-Ethyliden, 1,1-oder 2,2- Propyliden oder 1,1- oder 2,2-Butyliden, steht,
A₅ geradkettiges oder verzweigtes C₁-C₄-Alkylen, wie Methylen, 1,2-Ethylen, 1,3- oder 1,2-Propylen oder 1,4-, 1,3- oder 2,3-Butylen, oder eine direkte Bindung darstellt,
n für 0 steht,
R₁ und R₂ unabhängig voneinander Wasserstoff, C₁-C₄-Alkoxy, wie Methoxy, C₁-C₄-Alkyl, wie Methyl, Ethyl, Propyl, Isopropyl, Butyl, Isobutyl, Sekundärbutyl oder Tertiärbutyl, Trifluormethyl oder Halogen der Atomnummer bis und mit 35, wie Chlor oder Fluor, ferner Brom, bedeuten,
R₃ Wasserstoff bedeutet,
R₄ Carboxy, C₁-C₄-Alkoxycarbonyl, wie Methoxycarbonyl, Ethoxycarbonyl, Propyloxycarbonyl, Isopropyloxycarbonyl, Butyloxycarbonyl, Isobutyloxycarbonyl, Sekundärbutyloxycarbonyl, oder Tertiärbutyloxycarbonyl, unsubstituiertes oder durch C₁-C₄-Alkyl, wie Methyl, C₁-C₄-Alkoxy, wie Methoxy, Hydroxy, Halogen der Atomnummer bis und mit 35, wie Chlor, und/oder Trifluormethyl substituiertes Phenyl-C₁-C₄-alkoxycarbonyl, wie Benzyloxycarbonyl oder 1-Phenylethoxycarbonyl, Carbamoyl, N-C₁-C₇-Alkylcarbamoyl, vorzugsweise N-C₁-C₄-Alkylcarbamoyl, wie Methylcarbamoyl, Ethylcarbamoyl, Propylcarbamoyl, Isopropylcarbamoyl, Butylcarbamoyl, Isobutylcarbamoyl, Sekundärbutylcarbamoyl oder Tertiärbutylcarbamoyl, unsubstituiertes oder durch C₁-C₄-Alkyl, wie Methyl, C₁-C₄-Alkoxy, wie Methoxy, Hydroxy, Halogen der Atomnummer bis und mit 35, wie Chlor, und/oder Trifluormethyl substituiertes Phenylcarbamoyl, N,N-Di-C₁-C₄-Alkylcarbamoyl, wie Dimethylcarbamoyl, Diethylcarbamoyl, Dipropylcarbamoyl, Diisopropylcarbamoyl oder Dibutylcarbamoyl, darstellt und
R₅ Wasserstoff, Carboxy, C₁-C₄-Alkoxycarbonyl, wie Methoxycarbonyl, Carbamoyl, N-C₁-C₄-Alkylcarbamoyl, wie Methylcarbamoyl, unsubstituiertes oder durch C₁-C₄-Alkyl, wie Methyl, C₁-C₄-Alkoxy, wie Methoxy, Hydroxy, Halogen der Atomnummer bis und mit 35, wie Chlor, und/oder Trifluormethyl substituiertes Phenylcarbamoyl, N,N-Di-C₁-C₄-Alkylcarbamoyl, wie insbesondere Dimethylcarbamoyl, Cyano, Hydroxy, C₁-C₄-Alkoxy, wie Methoxy, Ethoxy, Propyloxy, Isopropyloxy, Butyloxy, Isobutyloxy, Sekundärbutyloxy oder Tertiärbutyloxy, C₃-C₄-Alkenyloxy, wie Allyloxy oder Methallyloxy, oder C₃-C₄-Alkinyloxy, wie Propargyloxy, bedeutet,
oder ein Salz davon.

6. Eine Verbindung gemäß Anspruch 1 der Formel 1, worin
A₁ eine Gruppe der Formel >CH-A₄-R₄ (Ia) darstellt,
A₂ Methylen ist,
A₃ eine Gruppe der Formel >N-A₅-R₅ (Ic) bedeutet,
A₄ für Methylen steht,
n für 0 steht,
A₅ eine direkte Bindung darstellt,
einer der Reste R₁ und R₂ C₁-C₄-Alkyl, wie Methyl, Halogen der Atomnummer bis und mit 35, wie Chlor, Fluor oder Brom, oder Nitro und der andere Wasserstoff oder C₁-C₄-Alkyl, wie Methyl, Halogen der Atomnummer bis und mit 35, wie Chlor, Fluor oder Brom, bedeutet, R₃ Wasserstoff ist,
R₄ Carboxy, Phosphono, C₁-C₄-Alkoxycarbonyl, wie Methoxycarbonyl oder Ethoxycarbonyl, N-Hydroxycarbamoyl, N-C₁-C₄-Alkylcarbamoyl, wie Butyl- oder Tertiärbutylcarbamoyl, N,N-Di-C₁-C₄-alkylcarbamoyl, wie Dibutylcarbamoyl, N-(Carboxy-C₁-C₄-alkyl)carbamoyl, wie Carboxymethylcarbamoyl, N-(C₁-C₄-Alkoxycarbonyl-C₁-C₄-alkyl)carbamoyl, wie N-Methoxycarbonylmethylcarbamoyl oder N-Ethoxycarbonylmethylcarbamoyl, N-(Hydroxy-C₂-C₄-alkyl)carbamoyl, wie 2-Hydroxyethylcarbamoyl, N-C₁-C₄-Alkoxycarbamoyl, wie N-Tertiärbutyloxycarbamoyl, oder eine unsubstituierte oder durch C₁-C₄-Alkyl, wie Methyl, C₁-C₄-Alkoxy, wie Methoxy, Halogen der Atomnummer bis und mit 35, wie Chlor, Trifluormethyl, Nitro, Carboxy und/oder C₁-C₄-Alkoxycarbonyl, wie Methoxycarbonyl oder Ethoxycarbonyl substituierte N-Phenylcarbamoyl-, N-(Phenyl-C₁-C₄-alkyl)carbamoyl-, wie Benzylcarbamoyl- oder 2-Phenylethylcarbamoyl-, oder N-(Phenyl-C₁-C₄-alkoxy)carbamoyl-, wie N-Benzyloxycarbamoylgruppe bedeutet,
oder ein Salz davon.

7. Eine Verbindung gemäß Anspruch 1 der Formel I' 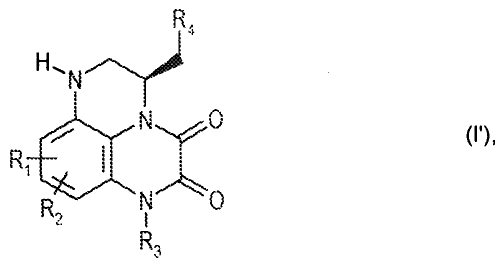 worin
einer der Reste R₁ und R₂ C₁-C₄-Alkyl, wie Methyl, Halogen der Atomnummer bis und mit 35, wie Chlor, Fluor oder Brom, oder Nitro und der andere Wasserstoff oder C₁-C₄-Alkyl, wie Methyl, Halogen der Atomnummer bis und mit 35, wie Chlor, Fluor oder Brom, bedeutet, R₃ Wasserstoff ist und
R₄ Carboxy, C₁-C₄-Alkoxycarbonyl, wie Methoxycarbonyl oder Ethoxycarbonyl, N-Hydroxycarbamoyl, N-C₁-C₄-Alkylcarbamoyl, wie Butyl- oder Tertiärbutylcarbamoyl, N,N-Di-C₁-C₄-alkylcarbamoyl, wie Dibutylcarbamoyl, N-(Carboxy-C₁-C₄-alkyl)carbamoyl, wie Carboxymethylcarbamoyl, N-(C₁-C₄-Alkoxycarbonyl-C₁-C₄-alkyl)carbamoyl, wie N-Methoxycarbonylmethylcarbamoyl oder N-Ethoxycarbonylmethylcarbamoyl, N-(Hydroxy-C₂-C₄-alkyl)carbamoyl, wie 2-Hydroxyethylcarbamoyl, N-C₁-C₄-Alkoxycarbamoyl, wie N-Tertiärbutyloxycarbamoyl, oder eine unsubstituierte oder durch C₁-C₄-Alkyl, wie Methyl, C₁-C₄-Alkoxy, wie Methoxy, Halogen der Atomnummer bis und mit 35, wie Chlor, Trifluormethyl, Nitro, Carboxy und/oder C₁-C₄-Alkoxycarbonyl, wie Methoxycarbonyl oder Ethoxycarbonyl substituierte N-Phenylcarbamoyl-, N-(Phenyl-C₁-C₄-alkyl)carbamoyl-, wie Benzylcarbamoyl- oder 2-Phenylethylcarbamoyl-, oder N-(Phenyl-C₁-C₄-alkoxy)carbamoyl-, wie N-Benzyloxycarbamoylgruppe bedeutet,
oder ein Salz davon.

8. Eine Verbindung gemäß Anspruch 1 der Formel I, worin
A₁ eine Gruppe der Formel >CH-A₄-R₄ (Ia) darstellt,
A₂ Methylen ist,
A₃ eine Gruppe der Formel >N-A₆-R₅ (Ic) bedeutet,
A₄ für Methylen steht,
n für 0 steht,
A₆ direkte Bindung darstellt,
R₁ und R₂ unabhängig voneinander Wasserstoff oder Halogen der Atomnummer bis und mit 35, wie Chlor, bedeuten,
R₃ Wasserstoff ist,
R₄ Carboxy, C₁-C₄-Alkoxycarbonyl, wie Methoxycarbonyl, Ethoxycarbonyl, Propyloxycarbonyl, Isopropyloxycarbonyl, Butyloxycarbonyl, Isobutyloxycarbonyl, Sekundärbutyloxycarbonyl oder Tertiärbutyloxycarbonyl, oder eine unsubstituierte oder durch C₁-C₄-Alkyl, wie Methyl, C₁-C₄-Alkoxy, wie Methoxy, Hydroxy, Halogen der Atomnummer bis und mit 35, wie Chlor, und/oder Trifluormethyl substituierte N-Phenylcarbamoylgruppe bedeutet und
R₅ Wasserstoff bedeutet,
oder ein Salz davon.

9. 8,9-Dichlor-2,4,5-trioxo-2,3,5,6-tetrahydro-1H,4H-1,3a,6-triaza-phenalen-3-ylessigsäureethylester;
8,9-Dichlor-2,4,5-trioxo-2,3,5,6-tetrahydro-1H,4H-1,3a,6-triaza-phenalen-3-ylessigsäurebenzylester;
8,9-Dichlor-2,4,5-trioxo-2,3,5,6-tetrahydro-1H,4H-1,3a,6-triaza-phenalen-3-ylessigsäure oder
8,9-Dichlor-2,4,5-trioxo-2,3,5,6-tetrahydro-1H,4H-1,3a,6-triaza-phenalen-3-yl-N-phenylacetamid oder jeweils ein Salz davon.

10. 8,9-Dimethyl-2,4,5-trioxo-2,3,5,6-tetrahydro-1H,4H-1,3a,6-triaza-phenalen-3-ylessigsäureethylester;
8,9-Dimethyl-2,4,5-trioxo-2,3,5,6-tetrahydro-1H,4H-1,3a,6-triaza-phenalen-3-ylessigsäure;
8,9-Dimethyl-2,4,5-trioxo-2,3,5,6-tetrahydro-1H,4H-1,3a,6-triaza-phenalen-3-ylessigsäure-N-phenyl-acetamid;
8,9-Dichlor-4,5-dioxo-2,3,5,6-tetrahydro-1H,4H-1,3a,6-triaza-phenalen-3-ylessigsäuremethylester;
8,9-Dimethyl-4,5-dioxo-2,3,5,6-tetrahydro-1H,4H-1,3a,6-triaza-phenalen-3-ylessigsäuremethylester;
8,9-Difluor-4,5-dioxo-2,3,5,6-tetrahydro-1H,4H-1,3a,6-triaza-phenalen-3-ylessigsäuremethylester;
4,5-Dioxo-2,3,5,6-tetrahydro-1H,4H-1,3a,6-triaza-phenalen-3-ylessigsäuremethylester;
8-Brom-4,5-dioxo-2,3,5,6-tetrahydro-1H,4H-1,3a,6-triaza-phenalen-3-ylessigsäuremethylester;
8-Trifluormethyl-4,5-dioxo-2,3,5,6-tetrahydro-1H,4H-1,3a,6-triaza-phenalen-3-ylessigsäuremethylester;
8,9-Dichlor-4,5-dioxo-2,3,5,6-tetrahydro-1H,4H-1,3a,6-triaza-phenalen-3-ylessigsäure;
8,9-Dimethyl-4,5-dioxo-2,3,5,6-tetrahydro-1H,4H-1,3a,6-triaza-phenalen-3-ylessigsäure;
8,9-Difluor-4,5-dioxo-2,3,5,6-tetrahydro-1H,4H-1,3a,6-triaza-phenalen-3-ylessigsäure;
4,5-Dioxo-2,3,5,6-tetrahydro-1H,4H-1,3a,6-triaza-phenalen-3-ylessigsäure,;
8-Brom-4,5-dioxo-2,3,5,6-tetrahydro-1H,4H-1,3a,6-triaza-phenalen-3-ylessigsäure;
8-Trifluormethyl-4,5-dioxo-2,3,5,6-tetrahydro-1H,4H-1,3a,6-triaza-phenalen-3-ylessigsäure;
8,9-Dichlor-4,5-dioxo-2,3,5,6-tetrahydro-1H,4H-1,3a,6-triaza-phenalen-3-yl-N-phenylacetamid;
8,9-Dimethyl-4,5-dioxo-2,3,5,6-tetrahydro-1H,4H-1,3a,6-triaza-phenalen-3-N-phenylacetamid;
8,9-Difluor-4,5-dioxo-2,3,5,6-tetrahydro-1H,4H-1,3a,6-triaza-phenalen-3-N-phenyl-acetamid;
4,5-Dioxo-2,3,5,6-tetrahydro-1H,4H-1,3a,6-triaza-phenalen-3-N-phenyl-acetamid;
8-Brom-4,5-dioxo-2,3,5,6-tetrahydro-1H,4H-1,3a,6-triaza-phenalen-3-phenyl-acetamid;
8-Trifluormethyl-4,5-dioxo-2,3,5,6-tetrahydro-1H,4H-1,3a,6-triaza-phenalen-3-phenylacetamid;
8,9-Dichlor-4,5-dioxo-2,3,5,6-tetrahydro-1H,4H-1,3a,6-triaza-phenalen-3-yl-N-benzylacetamid;
8,9-Dichlor-4,5-dioxo-2,3,5,6-tetrahydro-1H,4H-1,3a,6-triaza-phenalen-3-yl-N-(2-phenylethyl)-acetamid;
8,9-Dichlor-4,5-dioxo-2,3,5,6-tetrahydro-1H,4H-1,3a,6-triaza-phenalen-3-yl-N-(2-hydroxyethyl)-acetamid;
8,9-Dichlor-4,5-dioxo-2,3,5,6-tetrahydro-1H,4H-1,3a,6-triaza-phenalen-3-yl-N-(ethoxycarbonylmethyl)-acetamid;
8,9-Dichlor-4,5-dioxo-2,3,5,6-tetrahydro-1H,4H-1,3a,6-triaza-phenalen-3-yl-N-(carboxymethyl)-acetamid;
8,9-Dichlor-4,5-dioxo-2,3,5,6-tetrahydro-1H,4H-1,3a,6-triaza-phenalen-3-yl-N-(3-trifluormethylphenyl)-acetamid;
8,9-Dichlor-4,5-dioxo-2,3,5,6-tetrahydro-1H,4H-1,3a,6-triaza-phenalen-3-yl-N-(4-chlorphenyl)-acetamid;
8,9-Dichlor-4,5-dioxo-2,3,5,6-tetrahydro-1 H,4H-1,3a,6-triaza-phenalen-3-yl-N-(4-methylphenyl)-acetamid;
8,9-Dichlor-4,5-dioxo-2,3,5,6-tetrahydro-1H,4H-1,3a,6-triaza-phenalen-3-yl-N-(4-methoxyphenyl)-acetamid;
8,9-Dichlor-4,5-dioxo-2,3,5,6-tetrahydro-1 H,4H-1,3a,6-triaza-phenalen-3-yl-N-(4-phenoxyphenyl)-acetamid;
8,9-Dichlor-4,5-dioxo-2,3,5,6-tetrahydro-1H,4H-1,3a,6-triaza-phenalen-3-yl-N-(3-nitrophenyl)-acetamid;
8,9-Dichlor-4,5-dioxo-2,3,5,6-tetrahydro-1H,4H-1,3a,6-triaza-phenalen-3-yl-N-(4-ethoxycarbonylphenyl)acetamid;
8,9-Dichlor-4,5-dioxo-2,3,5,6-tetrahydro-1H,4H-1,3a,6-triaza-phenalen-3-yl-N-(4-trifluormethylphenyl)-acetamid;
8,9-Dichlor-4,5-dioxo-2,3,5,6-tetrahydro-1H,4H-1,3a,6-triaza-phenalen-3-yl-N-benzyloxyacetamid;
8,9-Dichlor-4,5-dioxo-2,3,5,6-tetrahydro-1H,4H-1,3a,6-triaza-phenalen-3-yl-N-tertiärbutyloxy-acetamid;
8,9-Dichlor-4,5-dioxo-2,3,5,6-tetrahydro-1H,4H-1,3a,6-triaza-phenalen-3-yl-N-(3-biphenyl)acetamid;
8,9-Dichlor-4,5-dioxo-2,3,5,6-tetrahydro-1H,4H-1,3a,6-triaza-phenalen-3-yl-N-(3-phenoxyphenyl)-acetamid;
8,9-Dichlor-4,5-dioxo-2,3,5,6-tetrahydro-1H,4H-1,3a,6-triaza-phenalen-3-yl-N,N-dimethylacetamid;
8,9-Dichlor-4,5-dioxo-2,3,5,6-tetrahydro-1H,4H-1,3a,6-triaza-phenalen-3-yl-N-butyl-acetamid;;
8,9-Dichlor-4,5-dioxo-2,3,5,6-tetrahydro-1 H,4H-1,3a,6-triaza-phenalen-3-yl-N-tertiärbutylacetamid;
8,9-Dichlor-4,5-dioxo-2,3,5,6-tetrahydro-1H,4H-1,3a,6-triaza-phenalen-3-yl-N,N-dibutylacetamid;;
8,9-Dichlor-4,5-dioxo-2,3,5,6-tetrahydro-1H,4H-1,3a,6-triaza-phenalen-3-yl-N-(adamantan-1-yl)-acetamid;
8,9-Dichlor-4,5-dioxo-2,3,5,6-tetrahydro-1H,4H-1,3a,6-triaza-phenalen-3-yl-N-methyl-acetamid;
7,8-Dichlor-4-{2-oxo-2-[4-(2-oxo-imidazolidin-1-yl)piperidin-1-yl]ethyl}-5,6-dihydro-1H,4H,1,3a,6-triaza-phenalen-2,3-dion;
8,9-Dichlor-4,5-dioxo-2,3,5,6-tetrahydro-1H,4H-1,3a,6-triaza-phenalen-3-yl-N-methyl-N-phenyl-acetamid;
8,9-Dichlor-4,5-dioxo-2,3,5,6-tetrahydro-1H,4H-1,3a,6-triaza-phenalen-3-yl-N-[2-(2-oxoimidazolidin-1-yl]ethyl]-acetamid;
8,9-Dichlor-4,5-dioxo-2,3,5,6-tetrahydro-1H,4H-1,3a,6-triaza-phenalen-3-yl-N-(2-morpholinoethyl)-acetamid;
9-Propionyl-4,5-dioxo-2,3,5,6-tetrahydro-1 H,4H-1,3a,6-triaza-phenalen-3-yl-N-phenylacetamid;
9-Nitro-4,5-dioxo-2,3,5,6-tetrahydro-1H,4H-1,3a,6-triaza-phenalen-3-ylessigsäuremethylester;
7-Nitro-4,5-dioxo-2,3,5,6-tetrahydro-1H,4H-1,3a,6-triaza-phenalen-3-ylessigsäuremethylester;
9-Nitro-4,5-dioxo-2,3,5,6-tetrahydro-1H,4H-1,3a,6-triaza-phenalen-3-ylessigsäure;
7-Nitro-4,5-dioxo-2,3,5,6-tetrahydro-1H,4H-1,3a,6-triaza-phenalen-3-ylessigsäure;
9-Nitro-4,5-dioxo-2,3,5,6-tetrahydro-1H,4H-1,3a,6-triaza-phenalen-3-yl-N-phenyl-acetamid,;
7-Nitro-4,5-dioxo-2,3,5,6-tetrahydro-1H,4H-1,3a,6-triaza-phenalen-3-yl-N-phenyl-acetamid;
1-Allyl-8-brom-4,5-dioxo-2,3,5,6-tetrahydro-1H,4H-1,3a,6-triaza-phenalen-3-ylessigsäuremethylester;
1-Allyl-8-brom-4,5-dioxo-2,3,5,6-tetrahydro-1H,4H-1,3a,6-triaza-phenalen-3-ylessigsäure;
1-Allyl-8-brom-4,5-dioxo-2,3,5,6-tetrahydro-1H,4H-1,3a,6-triaza-phenalen-3-y1-N-phenylacetamid;
8-Brom-1-(2,3-dihydroxypropyl)-4,5-dioxo-2,3,5,6-tetrahydro-1H,4H-1,3a,6-triaza-phenalen3-yl-N-phenyl-acetamid;
7,8-Dichlor-4-vinyl-5,6-dihydro-1H,4H-1,3a,6-triaza-phenalen-2,3-dion;
7,8-Dichlor-1H,6H-1,3a,6-triaza-phenalen-2,3,5-trion
oder
8,9-Dichlor-4,5-dioxo-2,3,5,6-tetrahydro-1 H,4H-1,3a,6-triaza-phenalen-3-ylmethanphosphonsäure
oder jeweils ein Salz davon.

11. Eine Verbindung gemäß einem der Ansprüche 1, 3, 4, 6, 7 und 10 zur Behandlung des menschlichen oder tierischen Körpers.

12. Eine Verbindung gemäß einem der Ansprüche 2, 5 und 9 zur Behandlung des menschlichen oder tierischen Körpers.

13. Pharmazeutische Präparate, enthaltend eine Verbindung gemäß einem der Ansprüche 1, 3, 4, 6, 7, 10 und 11 oder ein pharmazeutisch verwendbares Salz davon neben üblichen pharmazeutischen Hilfs- und Trägerstoffen.

14. Pharmazeutische Präparate, enthaltend eine Verbindung gemäß einem der Ansprüche 2, 5, 8, 9 und 12 oder ein pharmazeutisch verwendbares Salz davon neben üblichen pharmazeutischen Hilfs- und Trägerstoffen.

15. Verfahren zur Herstellung von neuen azaaliphatisch überbrückten Chinoxalin-2,3-dione Chinoxalin-2,3-dione der Formel I 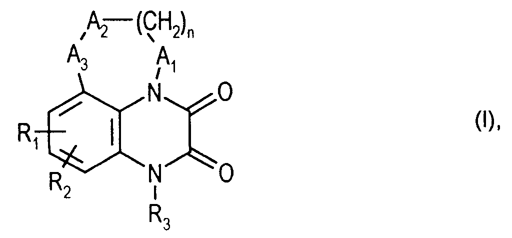 worin
A₁ Niederalkyliden oder eine Gruppe der Formel >CH-A₄-R₄ (Ia) darstellt,
A₂ Niederalkyliden oder eine Gruppe der Formeln >CH-A₄-R₄ (Ia) oder >C=O (Ib) oder >CH(OH)-A₅-R₄ (Ic) bedeutet,
A₃ eine Gruppe der Formel >N-A₆-R₅ (Id) darstellt,
A₄ für Niederalkylen steht,
A₅ und A₆ unabhängig voneinander Niederalkylen oder eine direkte Bindung darstellen,
n für 0 oder 1 steht,
R₁ und R₂ unabhängig voneinander Wasserstoff, gegebenenfalls durch Niederalkyl und/oder Niederalkanoyl substituiertes Amino, Nitro, Niederalkanoyl, gegebenenfalls verethertes Hydroxy, gegebenenfalls verestertes Carboxy, Carbamoyl, Cyano, gegebenenfalls halogeniertes Niederalkyl oder Halogen bedeuten,
R₃ Wasserstoff oder Hydroxy bedeutet,
R₄ gegebenenfalls verestertes oder amidiertes Carboxy, gegebenenfalls verestertes Phosphono oder 5-Tetrazolyl darstellt und
R₅ Wasserstoff, Niederalkyl, Niederalkenyl, Niederalkinyl, Aryl, Niederalkanoyl, gegebenenfalls verestertes oder amidiertes Carboxy, Cyano, 5-Tetrazolyl, gegebenenfalls verethertes oder verestertes Hydroxy oder Dihydroxyniederalkyl bedeutet,
und ihrer Salze, dadurch gekennzeichnet, daß man
eine Verbindung der Formel II 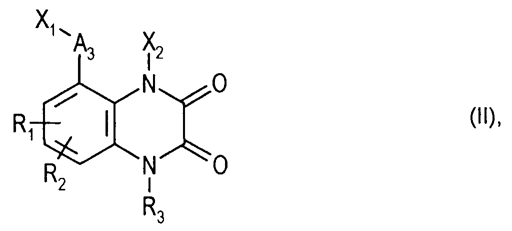 worin
X₁ eine Gruppe der Formel -A₂-(CH₂)ₙ-A₁-Y₁ (IIa) oder-A₂-(CH₂)ₙ-CH=A₄-R₄ (IIb) darstellt, wobei
Y₁ eine nukleofuge Abgangsgruppe bedeutet,
intramolekular cyclisiert und gewünschtenfalls eine erhaltene Verbindung in eine andere Verbindung der Formel I überführt, ein verfahrensgemäß erhältliches Isomerengemisch in die Komponenten auftrennt und das jeweils bevorzugte Isomere abtrennt und/oder eine verfahrensgemäß erhältliche freie Verbindung in ein Salz oder eine verfahrensgemäß erhältliches Salz in die entsprechende freie Verbindung überführt.

16. Verwendung einer Verbindung gemäß einem der Ansprüche 1 bis 11 zur Herstellung eines Arzneimittels für die Behandlung von pathologischen Zuständen, die auf Glycinantagonistische Blockierung von NMDA-sensitiven Rezeptoren ansprechen.

17. Verwendung gemäß Anspruch 16 zur Herstellung eines Arzneimittels für die Behandlung von neurodegenerativen Erkrankungen im Gefolge von Schlaganfall, Hypoglykämie, Anoxie oder Hirnlähmungserscheinungen, der cerebralen Ischämie, der cerebralen Ischämie bei Herzchirurgie oder Herzstillstand, perinataler Aphyxie, epilepstischer Anfälle, chorea Huntington, Alzheimer'scher und Parkinson'scher Erkrankung, amyotroper Lateralsklerose, Rückermark- und Himtrauma sowie Vergiftungserscheinungen durch Neurotoxine oder Suchtmittelmissbrauch, von ischämischen Erkrankungen des Auges, von Gefäß- und Muskelspasmen, von Migräne oder von lokaler oder generaler Spastizität, Konvulsionen, der Epilepsie, von Angst- und Schmerzzuständen und Trigeminusneuralgien.
